# EUROPEAN PATENT SPECIFICATION

(11) **EP 1 926 819 B1**
(45) Date of publication and mention of the grant of the patent: **16.01.2013**
(21) Application number: 06792110.6
(22) Date of filing: 15.09.2006
(51) Int. Cl.: C12N 15/82, C07K 14/08

(54) **PLANT VIRAL PARTICLES COMPRISING A PLURALITY OF FUSION PROTEINS CONSISTING OF A PLANT VIRAL COAT PROTEIN, A PEPTIDE LINKER AND A RECOMBINANT PROTEIN AND USE OF SUCH PLANT VIRAL PARTICLES FOR PROTEIN PURIFICATION**
PFLANZENVIRUSPARTIKEL MIT MEHREREN AUS EINEM PFLANZENVIRUS-HÜLLPROTEIN, EINEM PEPTID-LINKER UND EINEM REKOMBINANTEN PROTEIN BESTEHENDEN FUSIONSPROTEINEN SOWIE VERWENDUNG SOLCHER PFLANZENVIRUSPARTIKEL FÜR DIE PROTEINREINIGUNG
PARTICULES VIRALES VEGETALES COMPRENANT UNE PLURALITE DE PROTEINES DE FUSION CONSTITUEES D'UNE PROTEINE DE COQUE VIRALE VEGETALE, D'UN LIEUR PEPTIDIQUE, ET D'UNE PROTEINE RECOMBINANTE, ET UTILISATION DESDITES PARTICULES VIRALES VEGETALES POUR PURIFIER DES PROTEINES

(30) Priority: 17.09.2005 EP 05020311
(43) Date of publication of application: 04.06.2008
(73) Proprietor: Icon Genetics GmbH, 80333 München (DE)
(72) Inventor: WERNER, Stefan, 06110 Halle (DE); MARILLONNET, Sylvestre, 06126 Halle (DE); KLIMYUK, Viktor, 06114 Halle (DE); GLEBA, Yuri, 06114 Halle (DE)
(74) Representative: Blodig, Wolfgang
(86) International application number: PCT/EP2006/009029
(87) International publication number: WO 2007/031339

(56) References cited:
- EP-A- 1 162 267
- WO-A-02/068927
- US-A1- 2002 107 387
- O'BRIEN G J ET AL: "Rotavirus VP6 Expressed by PVX Vectors in Nicotiana benthamiana Coats PVX Rods and Also Assembles into Viruslike Particles" VIROLOGY, ACADEMIC PRESS,ORLANDO, US, vol. 270, no. 2, 10 May 2000 (2000-05-10), pages 444-453, XP004436314 ISSN: 0042-6822
- JELKMANN W ET AL: "A new potexvirus associated with strawberry mild yellow edge disease." THE JOURNAL OF GENERAL VIROLOGY. JUN 1990, vol. 71 ( Pt 6), June 1990 (1990-06), pages 1251-1258, XP009074270 ISSN: 0022-1317
- HIMMLER G ET AL: "DETECTION OF THE TRANS ACTIVITY OF THE PLUM POX VIRUS NIA-LIKE PROTEASE IN INFECTED PLANTS" JOURNAL OF GENERAL VIROLOGY, vol. 71, no. 7, 1990, pages 1623-1625, XP009074291 ISSN: 0022-1317
- SMOLENSKA L ET AL: "Production of a functional single chain antibody attached to the surface of a plant virus" FEBS LETTERS, ELSEVIER, AMSTERDAM, NL, vol. 441, no. 3, 28 December 1998 (1998-12-28), pages 379-382, XP004258968 ISSN: 0014-5793
- ARAI RYOICHI ET AL: "Conformations of variably linked chimeric proteins evaluated by synchrotron X-ray small-angle scattering." PROTEINS. 1 DEC 2004, vol. 57, no. 4, 1 December 2004 (2004-12-01), pages 829-838, XP002358153 ISSN: 1097-0134 cited in the application
- ARAI R ET AL: "Design of the linkers which effectively separate domains of a bifunctional fusion protein" PROTEIN ENGINEERING, OXFORD UNIVERSITY PRESS, SURREY, GB, vol. 14, no. 8, August 2001 (2001-08), pages 529-532, XP002334380 ISSN: 0269-2139 cited in the application

## Description

### FIELD OF THE INVENTION

The present invention relates to a process of affinity purifying a protein of interest that is an immunoglobulin using an affinity matrix comprising recombinant plant viral particles or recombinant plant virus-like particles. The invention further relates to the affinity matrix and to the recombinant viral particles, whereby the recombinant viral particles expose one or more recombinant proteins on their surface. The invention also relates to a fusion protein as a building block for said recombinant viral particles, to a polynucleotide encoding the fusion protein and to a plant, plant tissue or plant cells comprising said polynucleotide. The invention further relates to a process of producing said affinity matrix and to a process of producing said recombinant viral particles. The invention also relates to the use of said fusion protein for affinity purifying a protein of interest, that is an immunoglobulin .

### BACKGROUND OF THE INVENTION

It is known that in microbial protein expression systems that have been optimized with regard to product yield, up to 90% of total production costs are the costs related to purification of the protein of interest from the host, rather than expenses for the production itself. In order to make protein production more economical, strategies are needed that will allow rapid and inexpensive separation of the protein of interest or non-proteinaceous small molecule of interest, from other contaminating components .

It has been proposed (WO02/068927) to use plant viral particles having short peptides (e.g. a FLAG-tag) bound to the surface of plant viral particles for purification of a protein of interest by affinity purification. However, in the process of the prior art, the protein of interest to be purified has to be fused to a protein such as a single chain antibody or other purification tag that is capable of binding to the peptide bound to the surface of the viral particles. It is therefore necessary to cleave the protein to be purified after the affinity purification step, which represents an additional process step that one would like to avoid.

Antibodies and antibody derivatives constitute about 20% of biopharmaceutical products currently in development. The purification of antibodies accounts for 50-80% of the total production costs (for review: Roque et al., 2004, Biotechnol. Prog., 20:639-654). Protein A from *Staphylococcus aureus* is widely used as an affinity protein in processes of immunoglobulin purification (for review: Jungbauer & Hahn, 2004, Curr. Opin. Drug. Disc. & Dev., 7:248-256). Protein A reversibly interacts with the Fc domain of immunoglubulins (Lindmark et al., 1983, J. Immunol. Methods, 62:1-13; Gouda, et. al., 1998, Biochemistry, 37:129-136), predominantly via hydrophobic interactions (Dowd et al., 1998, Nat. Biotechnol., 16:190-195). The high stability and selectivity of protein A makes it a preferable generic ligand for immunoglobulin purification. The main source of protein A for the market has been recombinant protein A produced in *E. coli* (Duggleby & Jones, 1983, Nucleic Acids Res., 11:3065-3076; Engel et al., 1992, Protein Expr. Purif., 3:108-113). In prior art processes of purifying antibodies by affinity purification with protein A, protein A first has to be expressed and purified and then linked to a matrix such as sepharose that is then used for affinity purification of the antibodies. Thus, the production of the affinity matrix involves many steps and is laborious and expensive. Due to the costs for the affinity matrix, the affinity matrix is typically used for several purification runs, leading to a risk of contamination between consecutive samples purified on the same affinity matrix. A cheaper and readily producible affinity matrix for the purification of antibodies is therefore much needed. Such a cheap affinity matrix could be a single used matrix, avoiding the contamination risk.

It is therefore an object of the invention to provide a process of affinity purifying a protein of interest that is an immunoglobulin, wherein no cleavage step has to be performed on the protein of interest after the affinity purification step. It is a further object of the invention to provide a process of purifying immunoglobulins such as therapeutic antibodies or fusion proteins thereof using an economical and easily accessible affinity matrix. It is another object of the invention to provide a process of purifying therapeutic antibodies without the risk of contamination with human or other animal pathogens. It is a further object of the invention to provide an affinity matrix for said purifying processes.

### GENERAL DESCRIPTION OF THE INVENTION

These objects are achieved by a process of purifying a protein of interest that is an immunoglobulin, comprising the following steps:
(a) providing an affinity matrix comprising a fusion protein comprising the following fusion protein domains:
   (i) a plant viral coat protein,
   (ii) a recombinant protein comprising at least 50 amino acid residues as defined in claim 1,
   (iii) at least one peptide linker linking said plant viral coat protein and said recombinant protein, as defined in claim 1,
   said recombinant protein of said fusion protein having affinity to said protein of interest to be purified,
(b) contacting said affinity matrix with a liquid composition containing said protein of interest under conditions allowing binding of said protein of interest to said recombinant protein of said affinity matrix, whereby said affinity matrix is insoluble under said conditions,
(c) removing components of said liquid composition that have not bound to said recombinant protein from the mixture of step (b) under conditions preserving binding of said protein of interest to said recombinant protein of said affinity matrix, and
(d) separating said protein of interest from said affinity matrix to obtain said protein of interest in purified form.

The above objects are further achieved by recombinant viral particles or recombinant plant virus-like particles comprising a plurality of fusion protein molecules, said fusion protein comprising the following fusion protein domains:
(i) a plant viral coat protein domain,
(ii) a recombinant protein domain as defined in claim 4,
(iii) a peptide linker linking said plant viral coat protein and said recombinant protein, and comprising at least 10 amino acid residues,
wherein formation of said recombinant viral particle does not require free viral coat protein. Said recombinant domain or said coat protein domain may in turn have one or more than one domain.

The invention further provides recombinant viral particles or recombinant plant virus-like particles comprising fusion protein molecules, said fusion protein comprising the following fusion protein segments:
(i) a plant viral coat protein,
(ii) a recombinant protein comprising at least 50 amino acid residues as defined in claim 5 and
(iii) a peptide linker linking said plant viral coat protein and said recombinant protein, said peptide linker comprising at least 10 amino acid residues,
wherein said viral particle comprises at most 20 mol-% of free viral coat protein.

The invention further provides recombinant viral particles or recombinant plant virus-like particles comprising fusion protein molecules, said fusion protein comprising the following fusion protein segments:
(i) a plant viral coat protein of turnip vein clearing virus or a protein having an identity of at least 40 % to the coat protein of turnip vein clearing virus,
(ii) a recombinant protein comprising at least 50 amino acid residues and
(iii) a peptide linker linking said plant viral coat protein and said recombinant protein, said peptide linker comprising at least 10 amino acid residues.

The invention further provides recombinant viral particles or recombinant plant virus-like particles comprising fusion protein molecules, said fusion protein comprising the following fusion protein segments:
(i) a plant viral coat protein,
(ii) a recombinant protein comprising at least 50 amino acid residues and
(iii) a peptide linker linking said plant viral coat protein and said recombinant protein, said peptide linker comprising at least 10 amino acid residues,
wherein said recombinant protein is capable of binding to immunoglobulins to the Fc region of immunoglobulins, wherein said recombinant protein is protein A or protein G or variants of protein A or protein G, respectively, whereby said variants are capable of binding to the Fc region of immunoglobulins.

Also described is a process of purifying a protein of interest, comprising the following steps:
(a) providing a plurality of said recombinant viral particles or recombinant virus-like particles comprising a fusion protein as defined above, said recombinant protein of said fusion protein having affinity to said protein of interest to be purified,
(b) contacting said recombinant viral particles or recombinant virus-like particles with a liquid composition containing said protein of interest under conditions allowing binding of said protein of interest to said recombinant protein of said affinity matrix, whereby said affinity matrix is insoluble under said conditions,
(c) removing components of said liquid composition that have not bound to said recombinant protein from the mixture of step (b) under conditions preserving binding of said protein of interest to said recombinant protein of said affinity matrix, and
(d) separating said protein of interest from said affinity matrix.

Said recombinant protein is also referred to herein as "affinity protein".

The invention also provides a use of the affinity matrix of the invention or of the recombinant viral particles or recombinant plant virus-like particles of the invention for purifying a protein of interest. The invention further provides a use of the fusion protein of the invention for purifying a protein of interest.

The affinity matrix of the invention comprises the recombinant viral particles or the recombinant plant virus-like particles of the invention. Said affinity protein has affinity to immunoglobulins or derivatives thereof, preferably said affinity protein is staphylococcal protein A or streptococcal protein G.

The invention further provides a process of producing recombinant plant viral particles or recombinant plant virus-like particles according to the invention, comprising expressing said fusion protein in a bacterium, in a plant, in plant tissue, or in plant cells. Further provided is a polynucleotide encoding the fusion protein defined above and a plant, plant tissue or plant cells comprising said polynucleotide, according to claim 22.

Moreover, viral material obtainable from the plant, the plant tissue or plant cells defined above, is described.

Further, fusion proteins are provided. Notably, a fusion protein is provided comprising the following fusion protein segments: a plant viral coat protein, a linker peptide, and a staphylococcal protein A or a derivative of protein A capable of binding immunoglobulins, whereby said fusion protein is capable of forming viral particles or virus-like particles, as defined by claim 21.

Further, a kit-of-parts is provided comprising a plant, plant tissue or plant cells and a polynucleotide as defined above.

Moreover, uses are provided.

The invention also provides an affinity matrix for affinity purification of a protein of interest such as immunoglobulins, said affinity matrix comprising recombinant viral particles or recombinant plant virus-like particles comprising fusion protein molecules as defined above, wherein said recombinant viral particles comprise at most 20 mol-% of free viral coat protein relative to the sum of free viral coat protein and said fusion protein present in said affinity matrix. "Free viral coat protein" is plant viral coat protein not fused to said recombinant protein of the invention.

The inventors of the present invention have found that immunoglobulins can be affinity purified using an affinity matrix comprising said recombinant viral particles or said recombinant plant virus-like particles having an affinity protein such as protein A or protein G bound to their surface. The inventors have found that it is possible to fuse a recombinant protein (such as protein A, protein G etc., or a derivative thereof) to plant viral coat protein without destroying the capability of the obtained fusion protein to assemble to viral particles. In one embodiment, the fusion protein of the invention is expressed in plant cells without co-expressing free viral coat protein. Viral particles assembled from said fusion protein have a high density of recombinant protein bound to the surface of said viral particles.

Before the present invention, there has not been a technology of producing plant viral particles comprising a recombinant protein on the surface of said particles as a fusion with plant viral coat protein, wherein the size of recombinant protein is not restricted to short peptides and wherein viral particle formation does not require providing viral coat protein in addition to the fusion protein of the viral coat protein and the recombinant protein. Here, the system of the invention is devoid of the limitations of the prior art: it does preferably not require co-expression of free viral coat protein for the assembly of viral particles that display a recombinant protein on their surface. Consequently, the density of recombinant protein on the surface of the viral particles is much higher than in the case of viral particles comprising a high amount of free viral coat protein.

The fusion protein of the invention is a continuous polypeptide with an N-terminal end and a C-terminal end.

The fusion protein comprises the following domains: a plant viral coat protein domain, a recombinant protein domain and at least one peptide linker linking said plant viral coat protein domain and said recombinant protein domain. Said recombinant protein may be present within the primary structure of the amino acid sequence of the coat protein, whereby the coat protein domain may be formed by two coat protein segments of the primary structure of the fusion protein. In this embodiment, said recombinant protein is linked to said coat protein by two peptide linkers, one peptide linker linking the N-terminal portion of said recombinant protein to the N-terminal segment of said coat protein, the second peptide linker linking the C-terminal portion of said recombinant protein to the C- terminal segment of said coat protein.

In another embodiment, the coat protein domain, the recombinant protein domain and one peptide linker are sequential segments in the primary structure of said fusion protein. In this embodiment, there are two possibilities for the sequence of the fusion protein segments (or domains) from the N-terminus to the C-terminus of the fusion protein: (i) said plant viral coat protein is located at the N-terminus of the fusion protein and is followed by said peptide linker followed by said recombinant protein that is located at the C-terminal end of the fusion protein; (ii) said recombinant protein is located at the N-terminus of the fusion protein and is followed by said peptide linker followed by said coat protein that is located at the C-terminal end of the fusion protein. Thus, in one embodiment of the invention, the fusion protein comprises one peptide linker.

In all these embodiments, the fusion protein may comprise further amino acid residues or sequence segments at the N-terminus, at the C-terminus or within said fusion protein. "Domain" and "segment" are used interchangeably herein.

Said plant viral coat protein may be derived from any plant virus listed below. In one embodiment, said plant viral coat protein is derived from a plant virus forming rod-shaped viral particles. "Being derived" means that the coat protein used in the fusion protein of the invention does not have to be identical to the natural coat protein of a plant virus. Instead, the coat protein used in the fusion protein may have additions, deletions, insertions or mutations relative to a natural coat protein of a plant virus. It is only necessary that the coat protein maintains its capability to form viral or virus-like particles under suitable conditions. In one embodiment, at most 20 amino acid residues of the natural plant viral coat protein are deleted and/or mutated. In another embodiment, at most 20 amino acid residues are inserted into the natural sequence of the plant viral coat protein of the plant virus from which the coat protein of the invention is derived.

Said coat protein may be derived from a plus-sense single-stranded RNA virus. Examples of plant viruses the coat protein of which may be used in the present invention include tobamoviruses such as tobacco mosaic virus (TMV), turnip vein clearing virus, potato virus X, potato virus Y and fragments or homologues thereof, provided said fragments or homologues are capable of forming viral particles or virus-like particles under suitable conditions. Preferably, the coat protein of the invention has a sequence identity of at least 40% to the coat protein of turnip vein clearing virus, to tobacco mosaic virus, potato virus X or potato virus Y. In another embodiment, said sequence identity is at least 50%; in a further embodiment, said sequence identity is at least 60%. In an important embodiment, said coat protein has a sequence identity to the coat protein of tobacco mosaic virus of at least 80%.

The recombinant protein of the invention is exposed on the surface of said recombinant viral particles. The inventors have found for the first time that it is possible to create recombinant viral particles having a recombinant protein exposed on the surface of said viral particles without being restricted to small peptides of less than 40 or even less than 20 amino acids. Therefore, the invention shows its full potential with recombinant proteins having a size of at least 50 amino acid residues. However, in one embodiment, said recombinant protein has a size of at least 70 amino acid residues; in a further embodiment, said recombinant protein has a size of at least 90 amino acid residues; in a still further embodiment, said recombinant protein has a size of at least 110 amino acid residues.

The recombinant viral particles or virus-like particles of the invention are plant viral particles in that the coat protein domain or segment of said fusion protein is derived from a plant virus. The viral particles of the invention are recombinant in that they are assembled from a coat protein that is part of the fusion protein of the invention. The recombinant viral particles of the invention are also referred to herein as "said viral particles".

Said recombinant protein functions as an affinity protein e.g. when a matrix of said viral particles is used for affinity purification of a protein of interest. Therefore, the terms "recombinant protein" and "affinity protein" are used interchangeably herein for a protein exposed on the surface of the viral particles of the invention. The recombinant protein of the invention is recombinant in that it is a segment or domain of the fusion protein of the invention.

For allowing affinity purification of a protein of interest using the viral particles of the invention, said recombinant protein has an affinity to the compound or protein of interest. Herein, a protein to be purified using the affinity matrix or the recombinant viral particles or virus-like particles, or the fusion protein of the invention is termed "protein of interest". The protein of interest to be purified is an immunoglobulin. Said recombinant protein has affinity to immunoglobulins or derivatives thereof such as therapeutic antibodies. The affinity to immunoglobulins or derivatives thereof is to the constant region of the immunoglobulins. Said recombinant protein may be staphylococcal protein A or a domain or derivative thereof having affinity to immunoglobulins. In another embodiment, said recombinant protein may be streptococcal protein G or a derivative thereof capable of binding immunoglobulins.

The peptide linker of the invention links said plant viral coat protein and said recombinant protein in the primary structure of said fusion protein. The peptide linker allows assembly of viral particles of said fusion protein despite of the presence of said recombinant protein that may have a size of at least 50 amino acid residues. Said peptide linker should be flexible. In one embodiment, said peptide linker has no secondary structure in order to be flexible. In another embodiment, said peptide linker forms a helix. Preferably, said peptide linker does not form a β-sheet. It belongs to the general knowledge of the skilled person to design peptides having a predetermined secondary structure or no secondary structure. For example, proline residues break helices and β-sheets. One may therefore include one or more proline residues into said peptide linker. Alternatively, said peptide linker may contain a large proportion of glycine residues, whereby highly flexible peptide linkers may be obtained.

Said peptide linker has at least 10 amino acid residues. In another embodiment, said peptide linker has at least 15 amino acid residues; in a further embodiment, said peptide linker has at least 20 amino acid residues; in a further embodiment, said peptide linker has at least 30 amino acid residues. The bigger the recombinant protein to be bound to the surface of said viral particle, the longer should the peptide linker be made. For example, if said recombinant protein has more than 200 amino acid residues, the peptide linker preferably has at least 25 amino acid residues.

In one embodiment, the length of said peptide linker is between 10 and 70 amino acid residues. In another embodiment, the length of said peptide linker is between 13 and 50 amino acid residues. In a further embodiment, the length of said peptide linker is between 16 and 30 amino acid residues.

In one embodiment, said recombinant protein and said peptide linker together have at least 60 amino acid residues. In another embodiment, said recombinant protein and said peptide linker together have at least 80 amino acid residues. In a further embodiment, said recombinant protein and said peptide linker together have at least 100 amino acid residues. In a further embodiment, said recombinant protein and said peptide linker together have at least 130 amino acid residues.

The viral particles or virus-like particles of the invention can be produced by expressing a polynucleotide encoding said fusion protein of the invention in a bacterial or plant host. Said plant host may be plant cells, plant tissue or entire plants. Apart from encoding said fusion protein, said polynucleotide will have regulatory elements required for the expression of said fusion protein in the chosen host. Upon expressing said polynucleotide, the viral particles of the invention generally assemble within host cells or may be assembled in vitro after isolating said fusion protein from the host cells under suitable conditions.

Said viral particles of the invention do preferably not require the presence of free viral coat protein for assembly. Therefore, in one embodiment, said fusion protein is expressed without co-expressing free viral coat protein. The fusion protein of the invention can, however, assemble to said viral particles in the presence of free coat protein. In one embodiment, said viral particles comprise at most 30 mol-% free viral coat protein, preferably at most 20 mol-%, most preferably at most 10 mol-% of free viral coat protein. The content of free viral coat protein in said viral particles may be determined by solubilizing said viral particles and performing mass spectroscopy such as MALDI or ESI mass spectroscopy for determining the molecular weights and the relative abundance of the proteinaceous components of said viral particles. Any viral RNA contained in said viral particles may either be removed before performing mass spectroscopy or the signal thereof may be neglected when determining the relative abundance of the proteinaceous components of said viral particles.

In a further embodiment, an SDS-PAGE is performed on the viral particles and stained by Coomassie or silver staining. The intensity of the band caused by free viral coat protein will be at most 20 %, preferably at most 10 % of the intensity of the band caused by said fusion protein, as determined by a commercial gel reader.

For the purpose of this invention, a viral particle or a virus-like particle is defined as an oligomeric particle comprising a plurality of viral coat protein molecules, a plurality of the fusion protein molecules of the invention, or of a mixture of viral coat protein molecules and said fusion protein molecules of the invention. Said particle typically has a size and shape as seen in electron microscopy similar as the size and shape of the viral particle of the wild-type virus from which said coat protein is derived. The sizes of the viral particles or virus-like particles as determined in electron microscopy as described in Analytical Biochem., 333 (2004) 230-235 is preferably at least 10 nm in the shortest dimension, more preferably at least 13 nm in the shortest dimension.

In one embodiment, the recombinant viral particles or virus-like particles are produced in plant cells or plants using plant viral vectors, whereby the coat protein open reading frame (ORF) of a natural plant virus is replaced by the ORF of the fusion protein of the invention. The use of plant viral vectors has the advantage that high amounts of the fusion protein of the invention is produced per host cell, since the plant viral coat protein is the most abundant protein expressed in host cells after infection with a plant virus or plant viral vector. Further, cell to cell movement or systemic movement of the viral vector may lead to spread of the viral vector and to a high number of plant cells expressing said fusion protein. Methods of expressing a protein such as the fusion protein of the invention using a viral vector are known in the art. In one embodiment, the viral vector is introduced into plant cells or cells of a plant as part of a binary vector using Agrobacterium-mediated transformation.

The invention also provides an affinity matrix for purifying the protein of interest. Said affinity matrix comprises a plurality of the viral particles or virus-like particles of the invention. In one embodiment, said viral particles or virus-like particles in said affinity matrix are not covalently cross-linked. In another embodiment, the viral particles or virus-like particles in said affinity matrix may be cross-linked by a cross-linking agent. Cross-linking agents that can be used for cross-linking the viral particles of the invention are known in the art. Examples for such cross-linking agents are glutaraldehyde or bis-succinimides. A cross-linked affinity matrix has improved mechanical properties and a higher molecular weight. Further, covalent cross-linking allows to render said viral particles infection-deficient, which increases the safety of a product purified using said affinity matrix.

For purifying a protein of interest, the affinity matrix of the invention may be filled into a column for affinity chromatography. Affinity chromatography may then be carried out according to conventional methods. In another embodiment, said protein of interest may be purified using said affinity matrix by a batch method (cf. example 4). In any event, the affinity matrix of the invention is used in a solvent that does not dissolve said affinity matrix or said viral particles of the affinity matrix. A suitable solvent is an aqueous solvent, preferably the solvent is water. Due to the high molecular weight and said insolubility of the affinity matrix, the affinity matrix can easily be separated (e.g. by sedimentation) from the soluble contaminants in a solution from which a protein of interest is to be purified.

A protein of interest to be purified according to the invention is typically present in dissolved form in an aqueous solution or dispersion further containing soluble or insoluble contaminants. An example of such a solution is a cell lysate or cell supernatant. Insoluble matter is typically first separated by filtration or centrifugation for obtaining a clear solution. The clear solution may then be contacted with the affinity matrix of the invention, whereby the protein of interest binds to the affinity protein of said viral particles. Next, the affinity matrix having bound protein of interest is separated from the solution that originally contained the protein of interest. After washing the affinity matrix having bound protein of interest, the protein of interest may be detached from the affinity matrix under suitable conditions, whereby a solution containing purified protein of interest is obtained. A protocol for purifying immunoglobulins using an affinity matrix comprising viral particles having bound protein A is given in the examples.

### BRIEF DESCRIPTION OF THE DRAWINGS

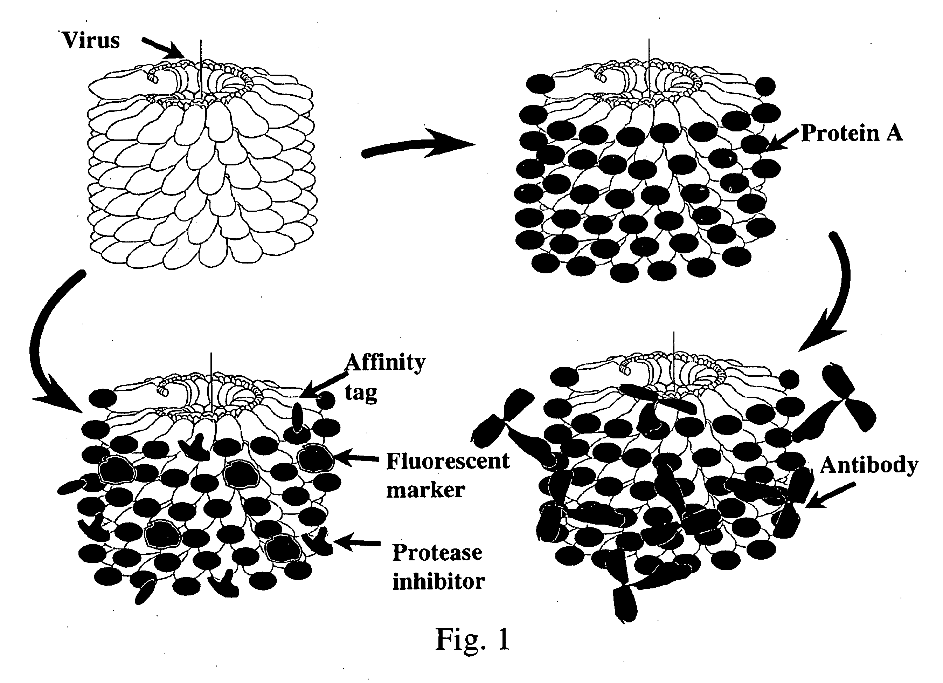

Figure 1 shows schematically at the top left side the structure of a plant viral particle. At the top right side, a viral particle according to the invention made up of the fusion protein of the invention is schematically shown, displaying protein A as said recombinant protein on the surface of the viral particle. At the bottom left, a viral particle according to the invention is shown and three different fusion proteins according to the invention, resulting in a viral particle displaying three different recombinant proteins on its surface, namely protein A, a fluorescent marker, and an affinity tag. If the viral particle shown at the top right side is used for affinity purification of the antibody, the viral particle binds antibody molecules via protein A.

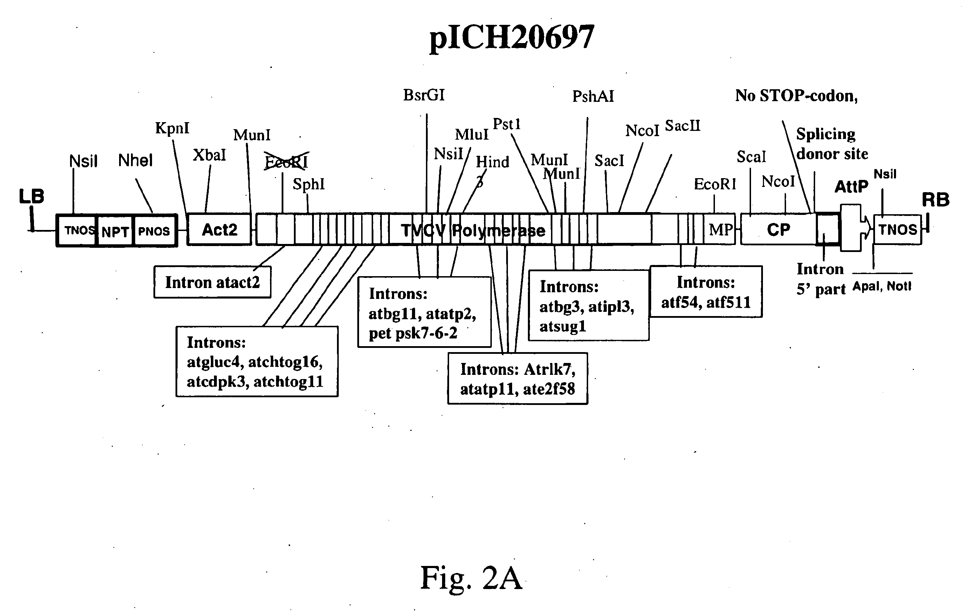

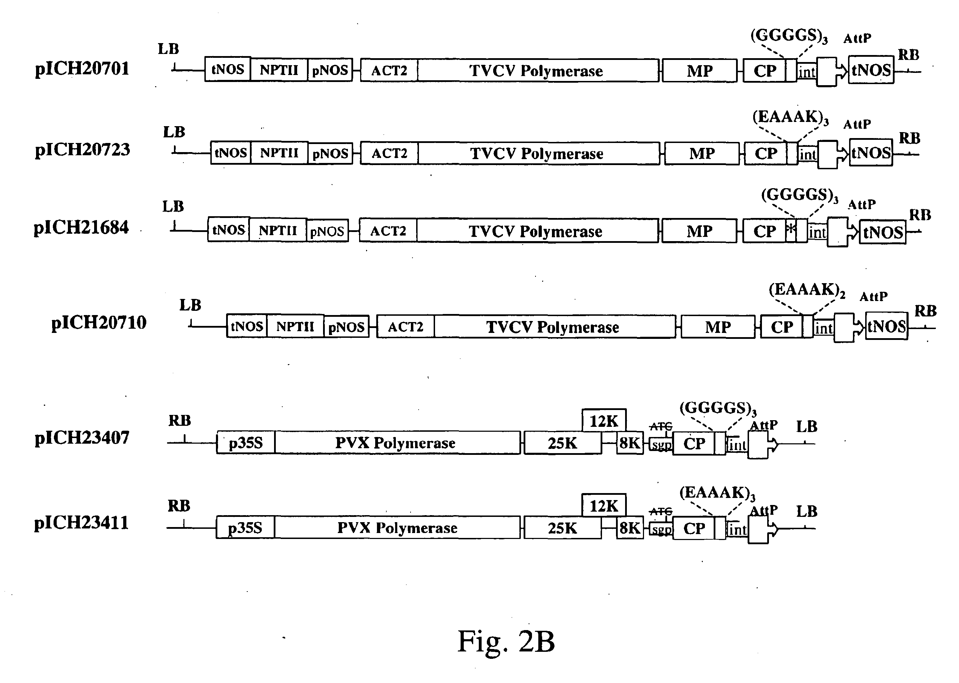

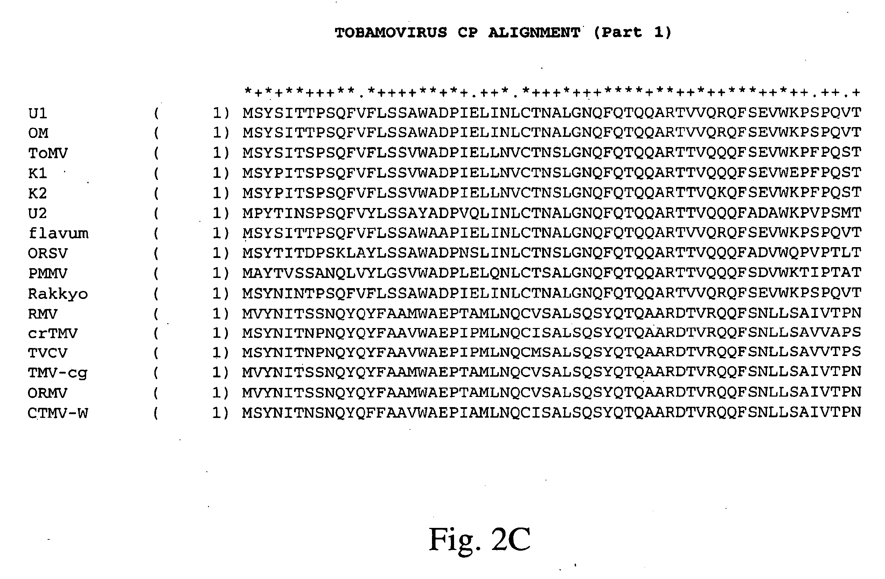

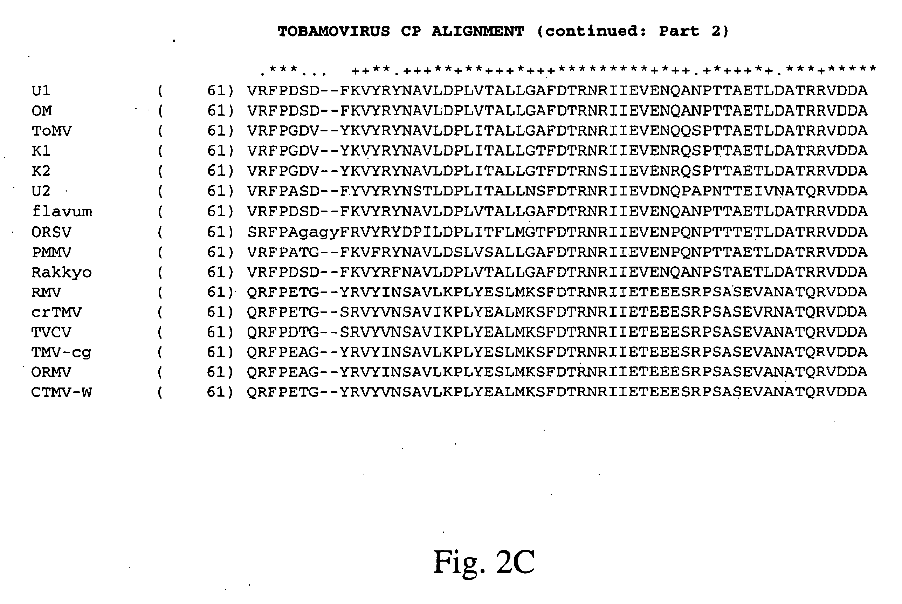

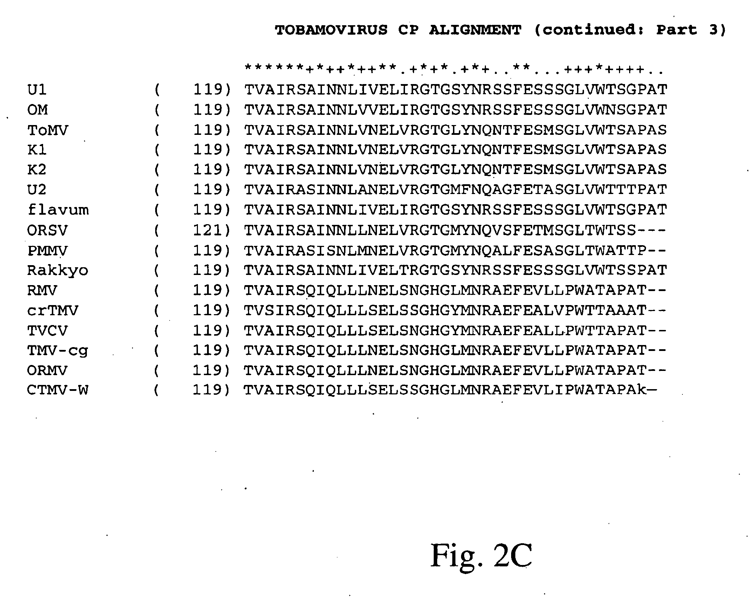

Figure 2 depicts T-DNA regions of different binary vectors.
   (A) shows pICH20697;
   (B) shows pICH20701, pICH20723, pICH21684, pICH20710, pICH23407 and pICH23411 containing 5' provectors of TMV-based and PVX-based viral expression systems (the intron positions are not shown but are identical to those of pICH20697 (Fig. 2A)).
      LB - left border of T-DNA; RB -right border of T-DNA; PNOS - promoter of agrobacterial nopaline synthase gene; TNOS - transcription termination region of agrobacterial nopaline synthase gene; NPTII - neomycin phoshotransferase II gene conferring resistance to kanamycin; P35S.- CaMV35S promoter; ACT2 - transcriptional enhancers of Arabidopsis actin2 gene promoter; MP - viral Movement protein; CP - viral coat protein; TVCV polymerase - RNA-dependent RNA polymerase of Turnip Vein Clearing Virus; PVX polymerase - RNA-dependent RNA polymerase of Potato Virus X; 25K, 12K, 8K - genes of triple gene block (TGB) region; sgp - subgenomic promoter; int - 5' part of intron sequence; AttP - recombination site recognised by site-specific integrase phiC31.
   (C) - Alignment of CP amino acid sequences from different tobamoviruses performed with the GeneBee program (http://www.belozersky.msu.ru) (courtesy of Prof. Y. Dorokhov). The meaning of signs at the top of the alignment is as follows: '^{.}' - the average weight of column pair exchanges is less than the weight matrix mean value; '.' - is less than mean value plus one SD; '+' - is less than mean value plus two SD; '*' - is more than mean value plus two SD. Gaps introduced for alignment are indicated by dashes. Data were taken from EMBL accession number J02415 for TMV U1 (U1: SEQ ID NO:19), X00052 for TMV common strain (OM: SEQ ID NO:20), X02144 for Tomato mosaic virus (ToMV: SEQ ID NO:21), AJ243571 for TMV Kazakh strain (K1: SEQ ID NO:22), Z92909 for TMV Kazakh strain (K2: SEQ ID NO:23), M34077 for Tobacco green mottle virus or strain (U2: SEQ ID NO:24), AF546184 for TMV flavum strain (SEQ ID. NO:25), AF321057 for Cucumber fruit mottle mosaic virus (CFMMV), D12505 for Cucumber green mottle mosaic virus (CGMMV), E04305 for Odontoglossum ringspot virus (ORSV: SEQ ID NO:26), AJ308228 for Pepper mild mottle virus (PMMV: SEQ ID NO:27), D63809 for TMV strain (Rakkyo: SEQ ID NO:28), J02413 for sunn-hemp mosaic virus (SHMV), AF254924 for ribgrass mosaic virus (RMV: SEQ ID NO:29), Z29370 for crucifer strain of TMV (crTMV: SEQ ID NO:30), U3387 for turnip vein-clearing virus (TVCV: SEQ ID NO:31), U30944 for crucifer strain of TMV (TMV-Cg: SECT ID NO:32), AB003936 for crucifer strain of TMV Wasabi (CTMV-W: SEQ ID NO:34), and Aguilar et al. (1996) for Oilseed rape mosaic virus (ORMV: SEQ ID NO:33).

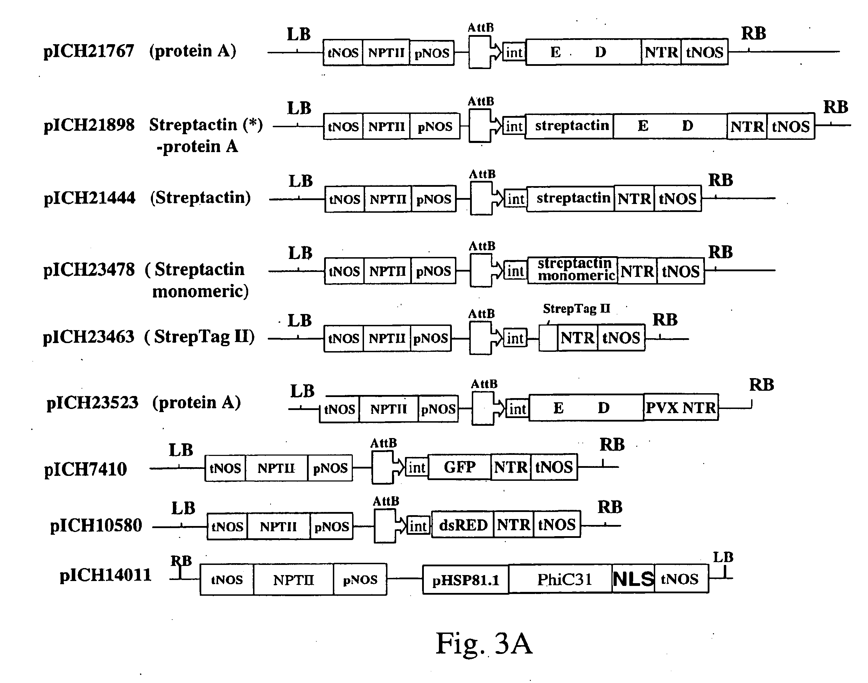

Figure 3A depicts T-DNA regions of binary vectors pICH21767, pICH21898, pICH21444, pICH323478, pICH23463, pICH23523, pICH7410, pICH10580 and pICH14011. LB - left border of T-DNA; RB-right border of T-DNA; PNOS - promoter of agrobacterial nopaline synthase gene; TNOS - transcription termination region of agrobacterial nopaline synthase gene; NPTII - neomycin phoshotransferase II gene conferring resistance to kanamycin; NTR - 3' non-translated region of viral RNA; AttB - recombination site recognised by site-specific integrase phiC31; pHSP81.1 promoter of gene encoding for Arabidopsis heat shock protein hsp81.1; NLS - nuclear localization signal; GFP - gene encoding synthetic green fluorescent protein; dsRED - red fluorescent protein; E, D - immunoglobulin binding domains E and D of *Staphylococcus aureus* protein A.

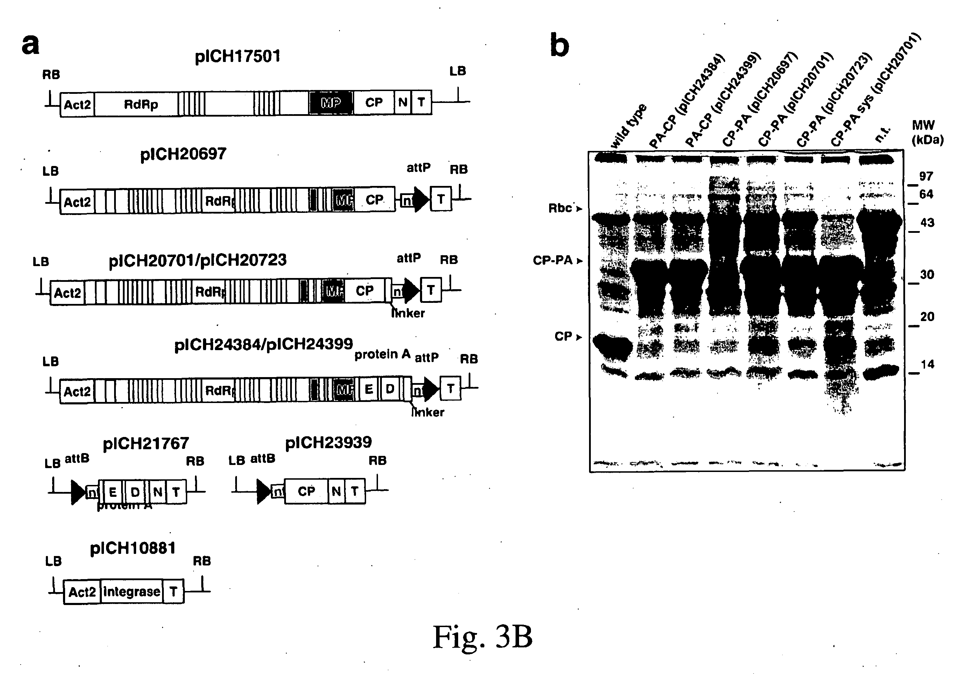

Figure 3B shows high-level expression of protein A-viral particle fusions. **(a)** Constructs used for transfection of *N. benthamiana* plants. Wild type CP is expressed from an assembled vector (pICH17501). CP-protein A fusions are expressed from separate 5'-and 3'-modules that are assembled *in planta* through a site-specific recombination catalyzed by an integrase (pICH10881). Short 15 aa linkers (hatched boxes) are included in the 5'-modules: a flexible linker (GGGGS)₃, in pICH20701 and pICH24384, and a helical linker (EAAAK)₃, in pICH20723 and pICH24399. pICH20697 does not contain any linker. White boxes represent introns for optimized expression. RdRp, RNA-dependent RNA polymerase; MP, Movement Protein; attP/attB, recombination sites; int, 5'- and 3'-part of intron for removal of the recombination site through splicing; N, 3'-non translated region; T, nos terminator. **(b)** Coomassie-stained polyacrylamide gel showing crude extracts from plants transfected with wild type virus, protein A N-terminal fusions with linkers (PA-CP), protein A C-terminal fusions (CP-PA) without (pICH20697) or with linkers (pICH20701/20723), C-terminal fusion with linker, systemic leaf, and non-transfected control. Rbc, Rubisco large subunit.

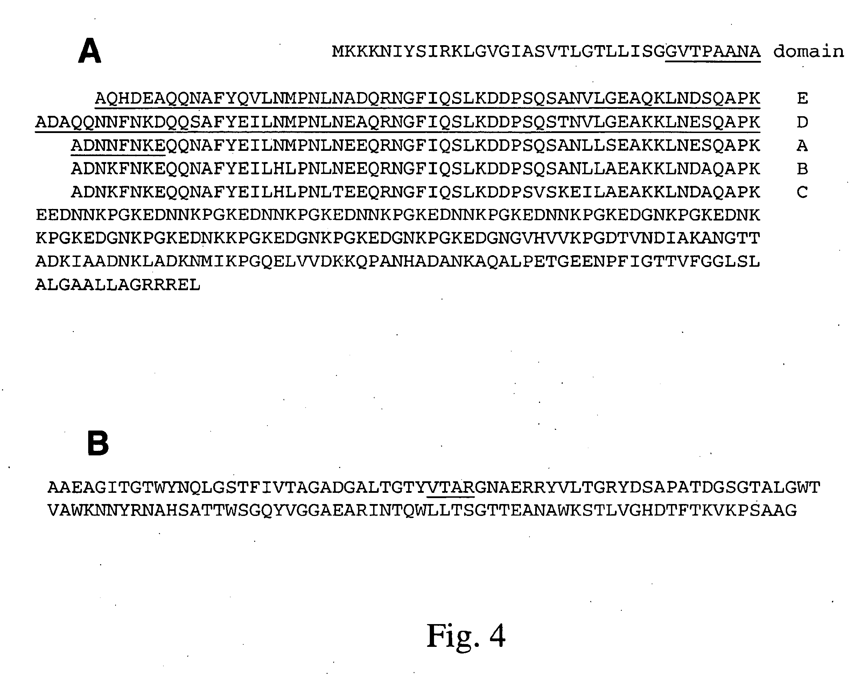

Figure 4 shows the sequences of (A) *Staphylococcus aureus* protein A (SEQ ID NO:35) and (B) a fragment of mature streptavidin (amino acid residues 12 - 139: SEQ ID NO:36).
   The part of protein A gene encoding for the underlined region of the protein sequence was re-synthesized with a codon usage optimized for expression in *N. tabacum* and for the structure and stability of the mRNA. It was cloned into TMV-3' provector (pICH21767).
   The length of the cloned sequence is 133 aa (domain E: 56 aa; domain D: 61 aa).
   The sequence fragment of mature streptavidin (aa 12-139) was cloned into the 3'-provector pICH21444 for fusion to CP; the mutated residues for increased affinity to StrepTagII are underlined (native sequence at this position is "ESAV").

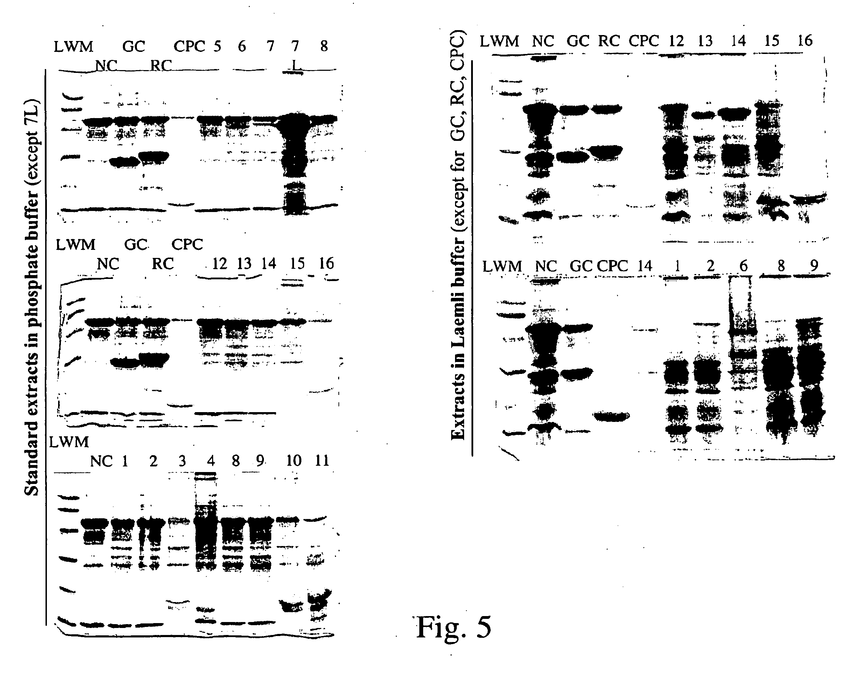

Figure 5 shows electrophoretic analysis of different fusions of recombinant protein with CP. Gels on the left: Standard extracts in phosphate buffer (except 7L). Gels on the right: Extracts in Laemli buffer (except for GC, RC, CPC)
   1) pICH20697-pICH7410 (GFP);
   2) pICH20697-pICH10580 (dsRED);
   3) pICH20697-pICH7410 (GFP), systemic leaves;
   4) 20697- pICH10580 (dsRED), systemic leaves;
   5) pICH20701;
   6) pICH20701- pICH7410 (GFP);
   7) pICH20701- pICH10580 (dsRED);
   8) pICH20710- pICH7410 (GFP);
   9) pICH20710- pICH10580 (dsRED;
   10) pICH20710- pICH7410 (GFP), systemic leaves;
   11) pICH20710- pICH10580 (dsRED), systemic leaves;
   12) pICH20723;
   13) pICH20723-pICH7410 (GFP);
   14) pICH20723- pICH10580 (dsRED);
   15) pICH20723- pICH7410 (GFP), systemic leaves;
   16) pICH20723- pICH10580 (dsRED), systemic leaves.
   NC - negative control; GC - GFP control; RC - dsRED control; CPC - CP control; L - Laemli buffer.
Figure 6
   (A) - Electron microscopy of recombinant viral particles. The bars represent 100 nm.
   (B) - Electrophoretic separation of protein extracted from inoculated leaf tissue.
      1) pICH20701-pICH21767 (protein-A, 2 domains);
      2) pICH20701-pICH21770 (protein-A, 1 domain);
      3) pICH20723-pICH21767 (2 domains);
      4) pICH20723-pICH21770 (1 domain);
      5) wild-type virus.
   (C) - Electrophoretic separation of protein extracted from recombinant viral particles.
      1) wild-type virus;
      2 - 7) pICH20701-pICH21767 (protein A, 2 domains), different preparations of viral particles;
      8) MW protein markers.
Figure 7. Measurement of antibody binding capacity of protein A displayed on the surface of plant virus-derived matrix. s - supernatant; p - pellet; HC - heavy chain of IgG; LC - light chain of IgG; CP-protA - viral coat protein-protein A fusion; MW - molecular weight protein markers (kDa).
Figure 8. Purification of IgG from plant extracts using viral particles displaying protein A on their surface.
   1) Crude extract;
   2) Supernatant after precipitation of IgG;
   3) Resuspended pellet after precipitation of IgG;
   4) Resuspended pellet after removal of particles;
   5) Resuspended pellet after PEG-precipitation of IgG (2x concentrated compared to other samples).
   HC - heavy chain of IgG; LC - light chain of IgG; CP-protA - viral coat protein-protein A fusion; MW - molecular weight protein markers (kDa).

### DETAILED DESCRIPTION OF THE INVENTION

Viral coat protein is the main building block of viral particles and virus-like particles (VLP). Viral particles and VLP are structured multimolecular biopolymers. By fusing a recombinant protein with a viral coat protein, it is possible to obtain viral particles with foreign epitopes (said recombinant protein) on their surface. The translational fusion of a recombinant protein with a viral coat protein (Hamamoto et al., 1993, BioTechnology, 11. 930-932; Gopinath et al., 2000, Virology, 267. 159-173; Porta et al., 1994, Virology, 202:949-955; Porta et al., 2003, Virology, 310: 50-63; JP6169789; US5977438; WO02068927) has, however, been restricted in the prior art, as the recombinant protein that could be fused to a plant viral coat protein has been limited to 20-25 amino acid residues (Turpen et al., 1995 Biotechnology, 13: 53-57; Sugiyama et al., 1995, FEBS Lett., 359: 247-250; US 2002/0107387). Speculations in the prior art such as in US 2002/0107387 that larger recombinant proteins could be fused to viral particles could not be confirmed experimentally and thus turned out to be wrong. The experiment shown in Fig. 3B shows that in the absence of specific measures according to the present invention, no fusion protein consisting of a viral coat protein and protein A is produced. Thus, US 2002/0107387 does not provide plant viral particles with recombinant proteins fused to the viral coat protein, whereby the recombinant protein comprises more than 50 amino acid residues.

Therefore, in the prior art, significant amounts (up to 95%) of free viral coat protein have been co-expressed with the fusion protein for co-assembly with the fusion protein, whereby viral particles consisting of up to 95% of free vial coat protein are obtained. Obviously, this limits any application of viral particles displaying a peptide on the surface.

In general, the limited size of peptides that could be fused to the CPs of plant viruses such as TMV, cowpea mosaic virus (CPMV), alfalfa mosaic virus, etc., while retaining the ability to assemble into functional virions has restricted, in the prior art, these systems to the expression of short immunogen epitopes and peptide hormones. Another publication (Santa-Cruz et al., 1996, Proc Natl Acad Sci U S A, 93:6286-6290) describes formation of Potato Virus X (PVX) virions containing on its surface green fluorescent protein (GFP) expressed as N-terminal fusion with potato virus X coat protein. However, as in the cases with TMV vectors, the expression of significant amount of wild type coat protein was necessary for assembly of viral particles. The latter was achieved either by expression of viral coat protein from independent vector stably integrated into plant chromosomal DNA or by using a cleavable linker peptide in GFP-CP fusion, thus providing a source for CP for virion formation. It was not possible to obtain viral particles displaying GFP on their surface in the absence of free viral coat protein. In a similar study, a single chain antibody was displayed on potato virus X viral particles containing free viral coat protein (Smolenska et al., FEBS Lett. 441 (1998) 379-382). Another work (Bendahmane et al., 2002, Proc. Natl. Acad. Sci. USA, 99:3645-3650) showed that fusion of GFP with coat protein of tobacco mosaic virus in the absence of a peptide linker does not produce recombinant viral particles.

There are many applications for which the recombinant viral particles of the invention are useful such as affinity chromatography. For example, purification of antibodies and antibody derivatives that constitute 20% of biopharmaceutical products currently in development, accounts for 50-80% of total manufacturing costs (for review: Roque *et al.,* 2004, *Biotechnol. Prog.,* 20:639-654). Protein A from *Staphylococcus aureus* is broadly used as affinity protein in the process of immunoglobulin purification (for review: Jungbauer & Hahn, 2004, Curr. Opin. Drug. Disc. & Dev., 7:248-256). Protein A reversibly interacts with the Fc domain of immunoglubulins (Lindmark et al., 1983, J. Immunol. Methods, 62:1-13; Gouda, et. al., 1998, Biochemistry, 37:129-136), predominantly via hydrophobic interactions (Dowd et al., .1998, Nat. Biotechnol., 16:190-195). The high stability and selectivity of protein A makes it a useful generic affinity protein for immunoglobulin purification. The main source of protein A for the market has been recombinant protein A produced in ***E.** coli* (Duggleby & Jones, 1983, Nucleic Acids Res., 11:3065-3076; Engel et al., 1992, Protein Expr. Purif., 3:108-113). Display of protein A as affinity protein on the surface of an affinity matrix, such as matrix comprising viral particles or virus-like particles opens the opportunity for a cheap source of an affinity matrix having bound protein A or having bound another immunoadsorbent to be used in downstream processing of recombinant monoclonal antibodies. Another protein that can be used in this invention is streptococcal protein G (Guss et al., 1986, EMBO J., 5: 1567-1575), that also has strong affinity to Fc domain of IgG (Sauer-Eriksson et al., 1995, Structure, 3:275-278) and also weak affinity to the Fab fragment (Derrick & Wigley, 1992, Nature, 359: 752-754).

The present invention utilizes various properties of plant viruses for the purposes of purifying and visualizing proteins of interest produced in different hosts (which for purposes of this invention is meant to include any biological protein production host or any non-biological protein production method). The general principle of the invention is shown in Figure 1: plant viral particles displaying one or more recombinant protein(s) on their surface and the use of said plant viral particles for the purification of a protein of interest (e.g. antibodies). Also, the present invention utilizes the ability of viral coat protein to polymerize and form highly organized protein structures. The definition "Viral particle" of the invention covers plant viral particles and plant virus-like particles (VLP) that contain a fusion protein comprising viral coat protein and a recombinant protein of interest in accordance with the claims of this invention. The terms "protein matrix" or "affinity matrix" mean a plurality of plant viral particles that together form a matrix comprising viral particles according to the invention. A rod-shaped viral particle is schematically shown in Figure 1. However, practically any sufficiently characterized plant virus can be adopted for practicing this invention. DNA and RNA viruses belonging to different taxonomic groups are suitable for constructing fusion protein comprising a plant viral coat protein.

A list of viruses to which this invention can be applied is presented below. Taxa names in quotes (and not in italic script) indicate that this taxon does not have an ICTV international approved name. Species (vernacular) names are given in regular script. Viruses with no formal assignment to genus or family are indicated):
**DNA Viruses: Circular dsDNA Viruses:** Family: *Caulimoviridae,* Genus: *Badnavirus, **type** species:* commelina yellow mottle virus, Genus: *Caulimovirus, **Type** species:* cauliflower mosaic virus, Genus "SbCMV-like viruses", *Type species:* Soybean chloroticmottle virus, Genus "CsVMV-like viruses", *Type species:* Cassaya vein mosaicvirus, Genus "RTBV-like viruses", *Type species:* Rice tungro bacilliformvirus, Genus: "Petunia vein clearing-like viruses", *Type species:* Petunia vein clearing virus; **Circular ssDNA Viruses:** Family: *Geminiviridae,* Genus: Mastrevirus (Subgroup I Geminivirus), *Type species:* maize streak virus. Genus: *Curtovirus* (Subaroup II Geminivirus), *Type species:* beet curly top virus, Genus: *Begomovirus* (Subaroup III Geminivirus). *Type species:* bean golden mosaic virus;

### RNA Viruses:

**ssRNA Viruses:** Family: *Bromoviridae,* Genus: *Alfamovirus, **Type species**:* alfalfa mosaic virus, Genus: *Ilarvirus, **Type species**:* tobacco streak virus, Genus: *Bromovirus, **Type species:*** brome mosaic virus, Genus: Cucumovirus, **Type species:** cucumber mosaic virus;
Family: *Closteroviridae,* Genus: *Closterovirus, **Type species:*** beet yellows virus, Genus: *Crinivirus, **Type species:*** Lettuce infectious yellows virus, Family: *Comoviridae,* Genus: *Comovirus, **Type species:** cowpea mosaic virus,* Genus: *Fabavirus, **Type species:*** broad bean wilt virus 1, Genus: *Nepovirus, **Type species:*** tobacco ringspot virus;
Family: *Potyviridae,* Genus: *Potyvirus, **Type species:*** potato virus Y, Genus: *Rymovirus, **Type species:** ryegrass mosaic virus,* Genus: *Bymovirus, **Type species:*** barley yellow mosaic virus;
Family: *Sequiviridae,* Genus: *Sequivirus, **Type species:*** parsnip yellow fleck virus, Genus: *Waikavirus, **Type species:*** rice tungro spherical virus; Family: *Tombusviridae,* Genus: *Carmovirus, **Type species:*** carnation mottle virus, Genus: *Dianthovirus, **Type species:*** carnation ringspot virus, Genus: *Machlomovirus, **Type species:*** maize chlorotic mottle virus, Genus: *Necrovirus, **Type species:*** tobacco necrosis virus, Genus: *Tombusvirus,* ***Type species*:** tomato bushy stunt virus, **Unassigned Genera of ssRNA viruses,** Genus: *Capillovirus, **Type species:*** apple stem grooving virus;
Genus: *Carlavirus, **Type species:*** carnation latent virus; Genus: *Enamovirus, Type species:* pea enation mosaic virus,
Genus: *Furovirus, **Type species:*** soil-borne wheat mosaic virus, Genus: *Hordeivirus, **Type species:*** barley stripe mosaic virus, Genus: *Idaeovirus, **Type species:*** raspberry bushy dwarf virus;
Genus: *Luteovirus, **Type species:*** barley yellow dwarf virus; **Genus:** *Marafivirus, Type species:* maize rayado fino virus; **Genus:** *Potexvirus, Type species:* potato virus X; Genus: *Sobemovirus, Type species:* Southern bean mosaic virus, Genus: *Tenuivirus, Type species:* rice stripe virus,
Genus: *Tobamovirus, Type species:* tobacco mosaic virus,
Genus: *Tobravirus, Type species:* tobacco rattle virus,
Genus: *Trichovirus, Type species:* apple chlorotic leaf spot virus; Genus: *Tymovirus, Type species:* turnip yellow mosaic virus; Genus: *Umbravirus, Type species:* carrot mottle virus;
**Negative ssRNA Viruses:** Order: *Mononegavirales,* Family: *Rhabdoviridae,* Genus: *Cytorhabdovirus, Type Species:* lettuce necrotic yellows virus, Genus: *Nucleorhabdovirus, Type* ***species*:** potato yellow dwarf virus;
**Negative ssRNA Viruses:** Family: *Bunyaviridae,* Genus: *Tospovirus, Type* ***species*:** tomato spotted wilt virus;
**dsRNA Viruses:** Family: *Partitiviridae,* Genus: *Alphacryptovirus, Type* ***species*:** white clover cryptic virus 1, Genus: *Betacryptovirus, Type species:* white clover cryptic virus 2, Family: *Reoviridae,* Genus: *Fijivirus, Type species:* Fiji disease virus, Genus: *Phytoreovirus, Type species:* wound tumor virus, Genus: *Oryzavirus,Type species:* rice ragged stunt virus;

### Unassigned Viruses:

Genome: *ssRNA,* **Species** Garlic viruses A,B,C,D, **Species** grapevine fleck virus, **Species** maize white line mosaic virus, **Species** olive latent virus 2, **Species:** ourmia melon virus, **Species** Pelargonium zonate spot virus.

### Sizes and shapes of selected viruses are as follows.

Rod shaped viruses - TMV: the virions have ≈300 nm in length and =18 nm in diameter; PVX (filamentous; usually flexuous; with a clear modal length): 515 nm long and 13 nm in diameter; Brome Mosaic Virus: 26 nm in diameter.

### Symmetry/shape - icosahedral

Alfalfa mosaic virus (Nucleocapsids bacilliform, or quasi-isometric elongated): 35 nm long (Tb) or 30 nm long; Ta that occurs either in bacilliform (Ta-b) or ellipsoidal (Ta-t) shape) with no clear modal length: 56 nm long (B); 43 nm long (M); 18 nm in diameter.

Preferred viruses are plant viruses having a single-stranded plus-sense RNA genome. Other preferred viruses are plant viruses having rod-shaped viral particles.

The viruses (tobacco mosaic virus and potato virus X) used in the examples were predominantly chosen because of the ready availability of well-established expression systems for said viruses (Donson et al., 1991, Proc Natl Acad Sci U S A, 88:7204-7208; Shivprasad et al., 1999, Virology, 255:312-323; Marillonnet et al., 2004, Proc Natl Acad Sci U S A, 101:6852-6857; Marillonnet et al., 2005, Nat Biotechnol., 23:718-723; Chapman, Kavanagh & Baulcombe, 1992, Plant J., 2:549-557; Baulcombe, Chapman & Santa Cruz, 1995, Plant J., 7:1045-1053; Angell & Baulcombe, 1997, EMBO J., 16:3675-3684) including the very recently developed system for expression of hetero-oligomeric proteins (EP Application No. 05 001 819.1; WO 2006/079546). Other plant viruses including DNA viruses also can be used for practicing this invention (for reviews please refer to: Mullineaux et al., 1992, Genetic Engineering in Plant Viruses, CRC Press Inc., pp187-215; Timmermans et al., 1994, Ann. Rev. Plant Physiol. Plant Mol. Biol., 45:79-112; Porta & Lomonossoff, 2002, Biotechnol. Genet. Engineering Rev., 19:245-291).

We have surprisingly found that (a) flexible peptide linker(s) (either unable to form secondary structure or capable of forming a helical secondary structure) allows overcoming size restrictions in generating translational fusion of recombinant protein with plant viral coat protein. Said linker peptide presumably removes or significantly reduces a negative effect of fusion partners on each other's functionality. The linker peptides used in this invention may be flexible peptide linkers such as (GGGGS)ₙ or helix-forming peptide linkers such as (EAAAK)ₙ, wherein n may be 2 - 5. The peptide linkers are segments of said fusion protein. The use of this type of peptide linkers in fusion proteins of different proteins and their effect on the function of fusion proteins is described (Arai et al., 2001, Protein Eng., 14:529-532; Arai et al., 2004, Proteins, 57:829-838). In example 1, we describe the design of constructs containing linker peptides with n=3 (see also Fig. 1). However, similar constructs with n=2 (not shown) were also made. We found that longer peptide linkers (n=3) have significant positive impact on the function of fusion partners: viral CP (to form viral particle) and recombinant protein (for example, protein A to bind immunoglobulins). Longer linkers (longer than 15 amino acid residues) may further minimize interference between the functions of the fusion partners. However, the length of linker peptides suitable for practicing this invention can be significantly longer than mentioned above (e.g. up to 25 amino acid residues) and might depend on the choice of recombinant protein to be fused to viral coat protein. The choice of the peptide linker for practicing this invention is not limited to the linkers described above. Many other types of linkers can be used in this invention. Typically, the linkers in multi-domain or multi-repeat proteins have little secondary structure, but there are other types of linkers that form helical structures (Ortiz et al., 2005, J. Mol. Biol., 349:638-647). Also, there is information on linker design in phage display technology (Maruyama et al., 1994, Proc. Natl. Acad. Sci. USA, 91:8273-8277; Turner et al., 1997, J. Immunol. Methods, 205:43-54; Castillo et al., 2001, J. Immunol. Methods, 257:117-122; Weiss et al., 2000, Protein Sci., 9:647-654; Mikawa et al., 1996, J. Mol. Biol., 262:21-30). Despite significant difference in structure and biology between phages and plant virus-based systems, the inventors have found that the general principles for the choice of the peptide linker can be applied for plant viral particles. It appears that distancing the recombinant protein from the viral coat protein via said peptide linker peptide reduces interaction between the coat protein and the recombinant protein on the fusion protein and, as a consequence, preserves their functions. A peptide linker chosen for practicing this invention can be tested for its suitability in this application using various programs predicting protein secondary structures (e.g. NNPREDICT, link http://www.cmpharm.ucsf.edu/∼nomi/ nnpredict.html) (Kneller, et al., 1990, J. Mol. Biol. 214: 171-182). For predicting secondary structure, the linker peptide shall be analyzed as integrated part of the fusion protein due to possible influence of flanking sequences (coat protein and recombinant protein of the invention) on its secondary structure. An alternative program for this purpose is PredictProtein at Heidelberg University, Germany (http://www.embl-heidelberg.de/predictprotein/predictprotein.html).

The recombinant plant viral particles or plant virus-like particles can be produced by expressing said fusion protein of the invention. In one embodiment, said fusion protein is expressed in plant cells or plants using plant viral vectors. In such plant viral vectors, the coat protein of the virus from which the viral vector is derived may be replaced by a polynucleotide encoding said fusion protein of the invention.

Plant viral vectors are efficient tools for transient high yield expression of recombinant proteins such as the fusion protein of the invention in plants (for review see: Porta & Lomonossoff, 1996, Mol. Biotechnol., 5, 209-221; Yusibov et al., 1999, Curr. Top. Microbiol. Immunol., 240, 81-94; Gleba et al., 2004, Curr Opin Plant Biol. 7:182-188; Gleba et al., 2005, Vaccine, 23:2042-2048). Viral vector-based expression systems offer a significantly higher yield of transgene product (such as the fusion protein of the invention) compared to plant nuclear transgenes. For example, the level of recombinant protein can reach 5-50% of the total cellular plant protein content, when expressed from a viral vector (Kumagai et al., 2000, Gene, 245, 169-174; Shivprasad et al., 1999, Virology, 255, 312-323; Marillonnet et al., 2004, Proc Natl Acad Sci U S A, 101:6852-6857; Marillonnet et al., 2005, Nat Biotechnol., 23:718-723). There are several published patents which describe viral vectors suitable for systemic expression of the fusion protein of the invention in plants (US5316931; US5589367; US5866785). In general, these vectors can express a foreign gene as a translational fusion with a viral protein (US5491076; US5977438), from an additional subgenomic promoter (US5466788; US5670353; US5866785), or from polycistronic viral RNA using IRES elements for independent protein translation (WO0229068). Other systems (WO2005049839) rely on agrobacteria for systemic delivery of viral replicon and do have significantly higher capacity for the size of a foreign gene compared to systemic viral vectors.

In example 2 we describe the production and analysis of viral particles displaying different recombinant proteins of different sizes on its surface. The electrophoretic analysis of different viral CP-recombinant protein fusions expressed with the help of a viral vector is shown in Fig. 5. It is evident from the results of said analysis that the majority of tested fusions showed a high expression level in infiltrated leaves and in some cases, recombinant viral vectors could move systemically. However, systemic movement may lead to the reversion to wild type vector. Therefore, in one embodiment of the invention, the CP-recombinant protein fusions are expressed in inoculated leaves using agrobacterium-mediated delivery of viral vectors or provectors (Marillonnet et al., 2004, Proc Natl Acad Sci U S A, 101:6852-6857; Marillonnet et al., 2005, Nat Biotechnol., 23:718-723). Out of seven different recombinant proteins expressed as fusions with CP according to the invention, six recombinant proteins were successfully expressed and isolated from the plant tissue in form of protein matrix. The activity of the recombinant proteins displayed on the surface of said viral particles was confirmed experimentally. It is also evident that no expression of recombinant proteins fused to CP directly, without linker peptide, was observed neither in inoculated nor in systemic leaves (lanes 1, 2, 3, 4, Fig. 5). High level of expression in inoculated leaves was achieved with the same recombinant proteins fused to CP via a peptide linker according to the invention (lanes 7L, 13, 14; Fig. 5).

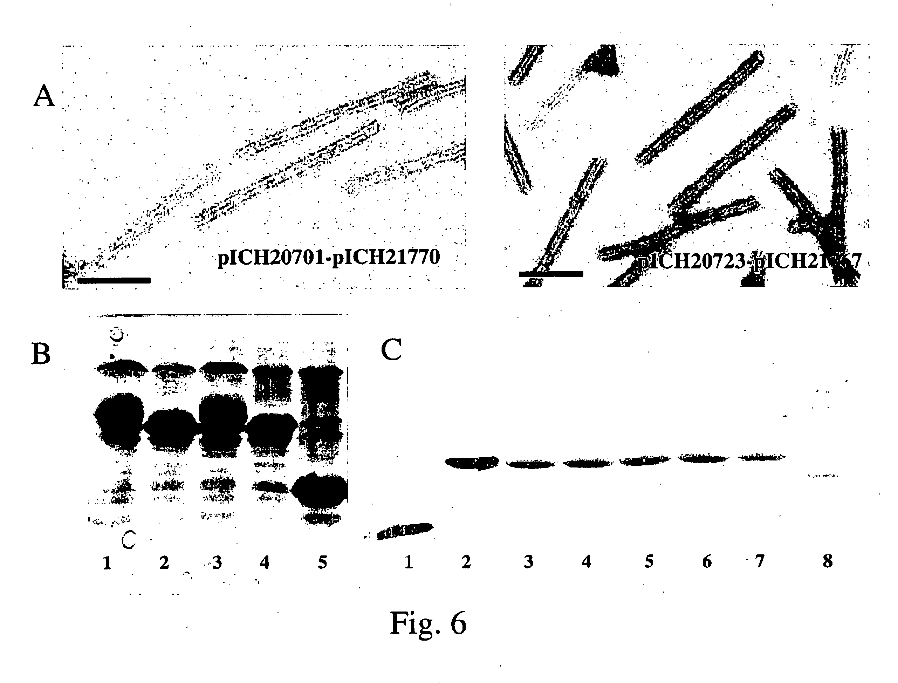

Another embodiment of this invention demonstrates the functionality of a recombinant protein displayed on the surface of a viral particle in biotechnology applications. We have chosen fusion proteins comprising the domains E and D of protein A (133 amino acid residues, see Fig. 4-A) and viral CP via a 15 amino acid peptide linker. Protein A is an efficient affinity tag broadly used in chromatographic purification of immunoglobulins, preferentially IgG and functional derivatives thereof (Fuglistaller, P., 1989, J. Immunol. Methods, 124-171-177; Fahmer et al., 1999, Biotechnol. Appl. Biochem., 30:121-128; Jungbauer & Hahn, 2004, Curr. Opin. Drug Discov. Dev., 7:248-256). There is a growing demand for recombinant protein A to be used in purification of recombinant immunoglobulins, as the number of monoclonal antibodies in clinical trials grows steadily, and for commercial production of antibodies large quantities of recombinant protein A will be required. Additionally, for pharmaceutical protein purification, single use reagents are a preferred choice. Currently, most of recombinant protein A is produced in *E. coli* (Hammond et al., 1990, Ann. NY Acad. Sci., 613:863-867; Engel et al., 1992, Protein Expr. Purif., 3:108-113; Cai et al., 1992, Chin. J. Biotechnol., 8:93-98). Our invention allows to produce large quantities of protein A, streptococcal protein G (Bond et al., 1993, J. Immunol. Methods, 166:27-33; Du et al., 2005, Biopolymers, 79:9-17; Honda et al., 1999, Biochemistry, 38:1203-1213; their active fragments, derivatives and mimics in large quantities, said recombinant proteins being already coupled to an affinity (or chromatographic) matrix, namely the plant virus derived protein matrix of viral particles. It is evident from Figure 6, that CP-protein A fusion is part of recombinant viral particles that may contain exclusively, within the detection limits of the Coomassie stained SDS-PAGE, fusion protein (Fig. 6B) and no detectable wild type CP as building block of the viral particle.

The surface of viral particles displaying protein A may serve as a high-affinity ligand suitable for purification of immunoglobulins. The relatively high molecular weight of virus particles allows their used as an affinity matrix and to develop simple procedures for purifying immunoglobulins that may be bound to the recombinant viral particles of the invention (or other protein of interest).

In addition, the recombinant viral particles can be further polymerized by cross-linking, yielding even higher molecular weight structures that are suitable for serving as an affinity matrix e.g. in protein purification procedures (Smith, Petrenko & Matthew, 1998, J. Immunol. Methods, 215:151-161). Another method of cross-linking viral particles can be by forming disulfide bridges between modified (cystein-added) coat proteins of different viral particles (Wang et al. 2002, Chem. Biol., 9: 813-819). This method also allows to inactivate viral particles, preventing viral vectors from replication. Additionally, various cross-linking agents can be used for inactivating viral particles, such as but not limited to formaldehyde (Barteling & Cassim, 2004, Dev. Biol., 119:449-455), ethyleneimine, N-acetylethyleneimine (Burrage et al., 2000, Vaccine, 18:2454-2461), UV irradiation (Freitas et al., 2003, J Virol Methods., 108:205-11) and other approaches.

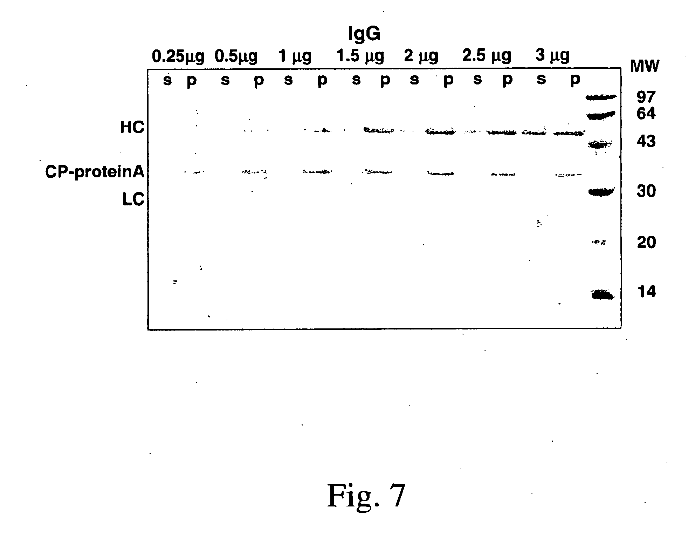

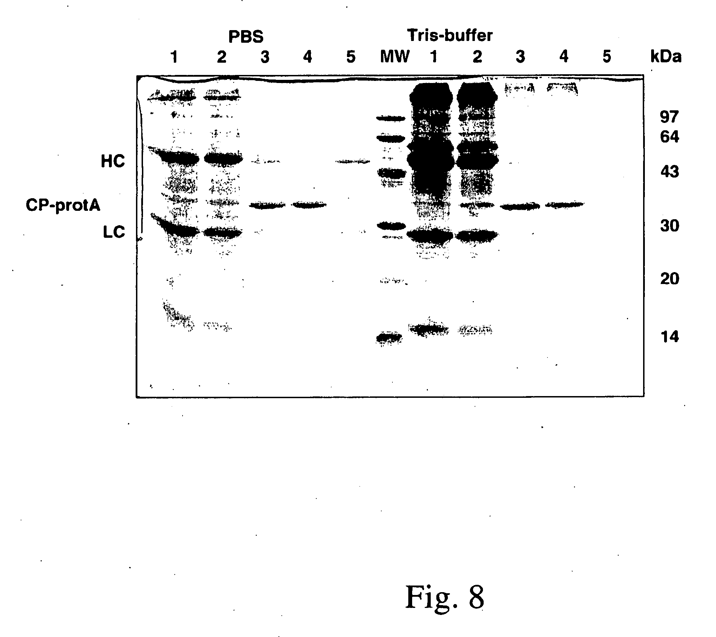

Viruses, whether naturally occurring wild-type or mutant viruses or genetically engineered viral vectors are self-replicating and as such are very inexpensive. Plant viral particles are also much larger than the great majority of proteins or small molecules for which purification procedures are required. The great difference in molecular weight or in physico-chemical properties can be effectively exploited to separate a protein or non-proteinaceous compound of interest from a mixture such as a tissue homogenate by binding the protein of interest to a virus particle according to the invention and then separating the resultant complex from the rest of the mixture. The association between the viral particle and the molecule of interest can later be dissolved in a number of ways known to those skilled in the art. In one embodiment of our invention, we demonstrate isolation of IgG from a plant extract using recombinant viral particles displaying IgG-binding domains on its surface. Viral particles displaying IgG binding domains of protein A were produced and isolated as described in Example 2. After evaluation of their binding capacity (Example 3, Figure 7), said particles were used for the purification of monoclonal antibodies (IgG class) produced in *Nicotiana benthamiana* plants agroinfiltrated with non-competing viral vectors (Example 4, Figure 8). It is evident from Figure 8, that a one-step purification using said particles produces an IgG sample with ca. 95% purity. The IgG purification protocol using said particles displaying protein A as fusion with viral coat protein is summarized in Table 1.

This invention also allows generating and utilizing recombinant viral particles having on their surface more than one (two or more) types of recombinant proteins, thus creating complex structures on the surface of plant viral particles. This may be achieved e.g. by using a recently developed plant virus-based expression system permitting to express more than one fusion of CP and a recombinant protein of interest with high yield (EP Application No. 05 001 819.1; WO 2006/079546). For example, two recombinant fusion proteins in roughly equimolar amounts can be expressed and assembled in viral particles using the invention. Alternatively, if different fusion proteins are required in a molar ratio other than equimolar, one of said fusion proteins could be expressed from a standard (e.g. driven by 35S promoter) expression cassette either transiently, or from a vector stably incorporated into plant chromosomal DNA, while the other fusion protein could be expressed from a viral vector. In yet another approach, viral particles can be reconstructed in vitro by mixing different recombinant viral particles in required proportions, deconstructing them by changing pH and/or ionic strength of the solution, and then reassembling them de novo, thus producing a different type of viral particles with different recombinant proteins on their surface. A schematic representation of a plant viral particle displaying more than one recombinant protein is shown on the left, bottom, of Figure 1. Such viral particle, in addition to having CP-protein A fusions, may also display a fluorescent marker (e.g. GFP or dsRed) helping to separate said viral particles or an affinity matrix thereof during a purification procedure. Additionally, a protease inhibitor as part of a fusion protein according to the invention may protect the protein of interest to be isolated (e.g. IgG) from proteolytic degradation.

Preferably, the viral particles of the invention are produced (expressed) in plants, as plants are practically free of human and animal pathogens, thus reducing the danger of infection by using viral particles isolated from viral or bacterial source. The cost of producing viral particles in plants, plant tissue or plant cells will be significantly lower compared to viral particles produced by an animal or bacterial source. In principle, however, the method may be practiced using a wide variety of host expression systems including plants (including cell and tissue cultures thereof), animals including non-human animal organisms, and animal and human cell cultures, fungi, bacteria and yeast.

The present method of purifying proteins of interest can be practiced in many different ways depending on several factors such as the nature of the protein of interest to be purified relative to the host and the manner in which the protein is produced in the host and the nature of the affinity between the virus particle and the protein. In embodiments where the protein of interest to be purified is produced endogenously (naturally) by a host, the host may be cultured and lysed. The lysate or a refined solution thereof containing the protein of interest may be contacted with the affinity matrix comprising the recombinant viral particles of the invention. Purification of proteins that are not produced endogenously by a host typically requires a genetic manipulation in order to supply the host with the machinery i.e., at least one transgene that encodes the protein of interest to be purified. In these embodiments, the transgene(s) may be introduced into a host as part of the viral expression/replication vector, or via a separate transformation event. The affinity of the recombinant protein displayed on the surface of the affinity matrix for the protein of interest produced by the host may be direct or indirect in the sense that the transgene may encode the protein of interest in the form of a fusion with a binding peptide that is recognized and bound by the affinity protein on the viral particles. Thus, the protein of interest may itself be a fusion protein.

In one embodiment, an exogenous (e.g., heterologous) protein of interest is expressed in a plant host (e.g., plant cells, tissue, homogenate or whole plant). This embodiment entails providing said viral particles displaying a recombinant protein as an affinity protein, wherein said recombinant protein has an affinity to the protein of interest to be purified. The conditions employed for dissociating the plant viral particle from the protein depends on the specific type of interactions and can be created by varying physico-chemical parameters e.g., pH; temperature; ions, chelating agents concentration, etc. Selecting appropriate conditions will be within the level of skill in the art of protein purification. Ultrafiltration is one such way of separating protein from the affinity matrix of said viral particles.

This invention is suitable for the purification of transgenic and endogenous proteins of interest alike

In the case of proteins that are exogenous to the host, transgenes encoding the protein of interest (by itself or in the form of a fusion with a peptide that binds the recombinant protein on the virus particle) are introduced into a non-human host in accordance with standard techniques. In general, these techniques may include stable or transient transformation or viral delivery (e.g., infection of the cell by the viral expression vector). Methods of creating transgenic organisms with stably integrated foreign genes are well described in the literature. For example, DNA can be transformed into plant cells via Agrobacterium-mediated delivery. See, U.S. Pat. Nos.: 5,591,616; 4,940,838; and 5,464,763. Other methods include particle or microprojectile bombardment (U.S. Pat. No. 5,100,792; European Patent (EP) 444,882 B1; EP 434,616 B1), microinjection (WO 09209696; WO 09400583 A1; EP 175,966 B1) and electroporation (EP 564,595 B1; EP 290,395 B1; WO 08706614 A1). Procedures of transgene delivery into animal, bacterial and yeast cells are well established. A popular method of transgene delivery into animal cells is retrovirus-mediated (Robbins & Givizzani, 1998; Reynolds et al., 1999). Other methods with synthetic (non-viral) carriers are also suitable (for review see: Bown et al., 2001). Transformation methods for yeast and bacterial cells are well described in many manuals e.g., Yeast Protocol Handbook (2000) and Sambrook et al., (1989).

The present invention is well amenable to industrial application and scaling-up because it can accommodate techniques such as tissue homogenization, centrifugation and ultrafiltration. It can be applied to production of proteins and small molecules in any prokaryotic or eukaryotic system. Thus, the invention represents a universal, inexpensive and scale-up method of purification of any protein of interest from any kind of prokaryotic or eukaryotic system.

The recombinant viral particles of the invention can be of interest for applications in many different areas - not only in biotechnology, but also in nanotechnology and molecular electronics applications. Plant viruses are very convenient for such purposes, as they are easy to produce and isolate and provide for high yield (up to 10 g of viral particles per kilogram of fresh tobacco leaves). Also, the viral particles (virions) can be purified industrially using simple 'low-tech' protocols (Creager et al, 1999, Plant Cell, 11:301-308)

### EXAMPLES

The following examples illustrate the invention further without limiting the scope of the invention.

### EXAMPLE 1

### Construction of TVCV- and PVX-based provectors for expression of CP-fusion proteins

The vectors used in the following examples are generally described in two recent publications (Marillonnet et al., 2004, Proc Natl Acad Sci U S A, 101:6852-6857; Marillonnet et al., 2005, Nat Biotechnol., 23:718-723). The cDNA for Potato Virus X (PVX) was generated from PVX isolate PV-0014 received from DSMZ collection (http://www.dsmz.de) by RT-PCR and used for creating PVX provectors. The descriptions of PVX-based expression system are provided in numerous publications (Chapman, Kavanagh & Baulcombe, 1992, Plant J., 2:549-557; Baulcombe, Chapman & Santa Cruz, 1995, Plant J., 7:1045-1053; Angell & Baulcombe, 1997, EMBO J., 16:3675-3684).

### a) The 5'-provectors of tobamovirus TVCV (Turnip Vein Clearing Virus)

The 3'-part of the TVCV Coat protein was amplified by PCR using primers *cptv1* and cpfus4 or *cpfus5* thus introducing a (GGGGS)₃-linker or a (EAAAK)₃-linker to the C-terminus of CP. The PCR products were cut with Ncol and Bsal and ligated into 5'-provector pICH20697 (Fig. 2A) containing the wild type CP without linker peptide resulting in constructs pICH20701 and pICH20723 (Fig. 2B). Vector pICH20697 by its intron structure is identical to that of (pICH18722) previously described (Marillonnet et al., 2005, Nature Biotechnol., 23:718-723).

### b) The 5'-provectors of PVX (Potato Virus X).

The CP with linkers was amplified by PCR with primers *pv5cptv* and *pv5p5r2* using pICH20701 or pICH20723 as template. PCR products were cloned as Nhel-Sacl fragments into PVX 5'-provector giving constructs pICH23407 and pICH23411 (Fig.2).

### c) Cloning the genes of interest in 3'-provectors

### Protein A

Protein A from *Staphylococcus aureus* contains five immunoglobulin (IgG) binding domains (Fig. 4-A). The first two of these domains (domains E and D) along with some additional amino acids on both sides (133 aa in total, Fig.4, underlined) were synthesized by GENEART (Regensburg, Germany). The sequence was optimized for expression in *Nicotiana tabacum.* The sequence was cloned as Bsal-Hindlll fragment into vector pICH10990 (Marillonnet et al., 2004, Proc Natl Acad Sci U S A, 101:6852-6857) giving construct pICH21767 (Fig. 3A). From this construct, the protein A sequence was transferred as HindIII-NdeI fragment into PVX 3'-provector pICH21799 resulting in pICH23523 (Fig. 3A). Construct pICH21770 (not shown) is similar to pICH21767, but contains only one IgG binding domain (domain E) of protein A.

### Reference proteins:

### Streptactin

Streptactin is a mutant form of streptavidin with increased affinity towards StrepTag II (Voss S. & Skerra A. 1997. *Prot Engin* **10**, 975-982). The 5'- and 3'-part of streptactin were amplified separately by PCR using primers *streppr1* and *streppr2* or *streppr3* and *streppr4* on genomic DNA from *Streptomyces avidinii* as template. PCR products encoding for protein fragment shown in Fig. 4-B, were cut with Bsal-Bpil (*streppr1* and *streppr2*) and BpiI-BamHI (*streppr3* and *streppr4*) and ligated into 3-provector pICH10990 (Marillonnet et al., 2004, Proc Natl Acad Sci U S A, 101:6852-6857) cut with BsaI-BamHI resulting in construct pICH21444 (Fig. 3A).

A mutant form of streptactin (V55T, T76R, L109T, V125R) that is supposed to be monomeric (Wu SC & Wong SL., 2005, J Biol Chem 280:23225-31) was engineered by site directed mutagensis with oligonucleotides *streppr5 -streppr12* leading to construct pICH23478 (Fig. 3A).

### StrepTag II

This tag was introduced into 3'-provector pICH21595 cut with Xbal, Bsal by adapter ligation with oligos *streptag5* and *streptag6.* The resulting construct was named pICH23463 (Fig. 3A).

### Other genes

A number of other genes (GFP, DsRed, antigens, cytokines) was cloned in a similar way into 3'-provectors (Marillonnet et al., 2004, Proc Natl Acad Sci U S A, 101:6852-6857) yielding in the constructs pICH7410 (GFP), pICH10580 (DsRED) (Fig. 3A).

### EXAMPLE 2

### Expression of CP-fusion proteins in plants

### Agroinfiltration

All constructs were electroporated into Agrobacterium tumefaciens GV3101. Agroinfiltrations of *N. benthamiana* plants were done essentially as described in Marillonnet et al., 2004, Proc Natl Acad Sci U S A, 101:6852-6857. Three agrobacterial strains containing 5' provector encoding CP, 3' provector encoding the recombinant protein and a source of a site-specific recombinase (pICH14011, Fig. 3A) for assembly of viral pro-vectors *in planta* via site-specific recombination into viral vector capable of amplification and expression of recombinant protein of interest were mixed together and used for infiltration. Small-scale infiltrations were done with a syringe; large-scale infiltrations were done using a vacuum device (Marillonnet et al., 2005, Nat Biotechnol., 23:718-723).

### Analysis of CP fusions expressed in N. benthamiana leaves

All recombinant protein fusions were extracted from infiltrated *N. benthamiana* leaves 6-11 days after infiltration and analysed by electrophoretic separation in polyacrylamide gels as previously described (Marillonnet et al., 2004, Proc Natl Acad Sci U S A, 101:6852-6857; Marillonnet et al., 2005, Nat Biotechnol., 23:718-723). The results of electrophoretic analysis of different CP-recombinant protein fusions are shown in Figure 5 (A,B). It is worth noticing that direct fusion (without linker peptide) of CP with recombinant protein of interest did not produce detectable expression.

Specifically, the use of pICH20697 5'-provector (Fig. 3B) in combination with appropriate 3' provector (pICH21767, Fig. 3B) did not provide any detectable expression. Also, electron microscopy analysis performed as described below did not detect any virus-like structures in the samples, unlike in case of using 5' provectors with linker peptide.

### Extraction and purification of viral particles

Infiltrated leaves were homogenized in 0.1 M K-phosphate buffer, pH 7.0 (2-3 ml buffer/g FW) using a leaf juice press or mortar and pestle. Insoluble matter was removed by filtration through miracloth. Leaf juice was treated once with one volume of chloroform and viral particles were precipitated with polyethylenglycol (PEG-6000) using standard procedures (Turpen & Reinl, 1998, Methods in Biotechnol., 3:89-101, eds. C. Cunningham & A.J.R. Porter, Humana Press, Totowa, NJ). Particles were resuspended in K-phosphate buffer and further purified by sucrose density centrifugation. The samples of viral particles containing CP fused with protein A fragment were analyzed by SDS-electrophoresis and by electronic microscopy. The samples for electron microscopy were prepared as described by Negrouk and colleagues (2004, Analytical Biochem., 333: 230-235). The results of analysis are shown in Figure 6.

### EXAMPLE 3

### Antibody binding capacity of protein A bound to the surface of plant viral particles

Aliquots (0.5 mg) of purified viral particles having bound recombinant protein A were mixed with different amounts of human IgG (Sigma I4506), incubated on ice for 1 hour and precipitated by centrifugation (10 min, 12.000 g). Pellets and supernatants were analysed by SDS-PAGE (Fig. 7). It was demonstrated that 1 mg of viral particles can bind up to 2 mg of IgG. The binding capacity is approx. 2 mg IgG/mg of viral particles (i.e. 1 molecule of IgG (150 kDa) is bound to every 3rd - 5th molecule of CP-protein A fusion (34 kDa) on the surface of recombinant viral particle.

### EXAMPLE 4

### Purification of antibodies expressed in plants using recombinant viral particles displaying protein A on their surface

Antibodies were expressed *in planta* using ICONs viral expression system for production of hetero-oligomeric proteins in plants (EP Application No. 05 001 819.1; WO 2006/079546). Leaf material containing monoclonal antibodies of the IgG class was ground in liquid nitrogen and extracted with 3 ml of PBS (Sambrook, Fritsch & Maniatis, 1989, Molecular Cloning: a Laboratory Manual, CSH, NY) per gram of fresh leaf weight (FW). Insoluble material was removed by two rounds of centrifugation (10 min, 16000 g). One hundred milligram of viral particles displaying protein A was added per one ml of plant extract and samples were incubated on ice for at least 1 hour. Antibodies bound to viral particles were precipitated by centrifugation (15 min, 12000 g) and resuspended in 0.25 volumes 0.1 M glycine pH 2.5. In order to remove viral particles, samples were adjusted to 1 % NaCl, 4 % PEG, incubated 30 min on ice and centrifuged 15 min at 10.000 g. Antibody-containing supernatants were transferred to fresh tubes, neutralized with 1/10 volume 1 M Tris/HCl pH 9.0 and adjusted to 14 % PEG by adding an appropriate volume of 25 % PEG-solution in PBS buffer. Samples were kept on ice for at least 1 hour and antibodies were precipitated by centrifugation (15 min, 16000 g). Summary of purification protocol is shown in Table 1. Anbtibodies were dissolved in a convenient volume of PBS and analyzed by gel-electrophoresis. An electrophoretic analysis of proteins from the purification procedure is shown in Figure 8.

**Table 1: Summary of IgG purification protocol using recombinant viral particles displaying protein A on its surface**

| | |
|---|---|
| 1. | Extract infiltrated leaves with 3 vol of PBS. Clear extract by 2x centrifugation. |
| 2. | Add viral particles (ca. 100 mg/ml) and incubate for 15-30 min on ice. |
| 3. | Centrifuge for 10 min, 12 000 g (11300 rpm in benchtop centrifuge), 4°C. |
| 4. | Discard supernatant, resuspend pellet in one volume of 0.1 M glycine, pH 2.5. |
| 5. | Add 1/10 volume of 12 % NaCl and 1/5 volume of 25 % PEG-6000 (in 0.1 M glycine, pH 2.5). Incubate on ice for 30 min. |
| 6. | Centrifuge for 15 min, 10 000 g (10300 rpm in benchtop centrifuge), 4°C. |
| 7. | Transfer supernatant to a fresh tube; neutralize with 1/10 volume of 1 M Tris/HCl pH 9.0; add 1 volume of 25 % PEG-6000 (in PBS) and incubate for 30 min on ice. |
| 8. | Centrifuge 15 min, 16 000 g (13000 rpm in benchtop centrifuge), 4°C. |
| 9. | Discard supernatant and centrifuge for additional 5 min. |
| 10. | Carefully remove all traces of supernatant; resuspend pellet in a convenient volume of PBS. |

### ANNEX 1

### Primer sequences:

| | |
|---|---|
| *cptv1:* | 5'-cggagctcttaatttaaaagaagaaaatgtcttacaac-3' (SEQ ID NO :1) |
| *cpfus4:* | 5'-tttggtctcatacctgagccaccgcctcctgatccaccgcctccacttcctccgcctc ctgtagcaggcgcagtagtcc-3' (SEQ ID NO :2) |
| *pv5cptv:* | 5'-cagctagcaacaaacaagaaatgtcttacaacattacaaacccg-3'(SEQ ID NO:3) |
| *pv5p5r2:* | 5'-gagctctctcgagcatgctacgcccccaactgagag-3' (SEQ ID NO:4) |
| *streppr1:* | 5'-gaagacaaaggtgctgctgaggctggaattacaggcacctgg-3' (SEQ ID NO:5) |
| *streppr2:* | 5'-gaagacaatgtaacataagttcctgtaagtgcaccatcggcgcc-3'(SEQ ID NO:6) |
| *streppr3:* | 5'-gaagacaatacagctagaggtaatgcagaaagacgttacgtcctg-3' (SEQ ID NO:7) |
| *streppr4:* | 5'-ggatccaaggtctcaacctgctgctgatggtttcaccttggtgaag-3' (SEQ ID NO:8) |
| *streppr5:* | 5'-tttggtctcagagtgtaacgtctttctgcattacc-3' (SEQ ID NO:9) |
| *streppr6:* | 5'-tttggtctcaactcttaccggtcgttacgacagc-3' (SEQ ID NO:10) |
| *streppr7:* | 5'-tttggtctcaccctccaaccgagggcggtgcc-3' (SEQ ID NO:11) |
| *streppr8:* | 5'-tttggtctcaagggtggcctggaagaataact-3' (SEQ ID NO:12) |
| *streppr9:* | 5'-tttggtctcagtgtccactgggtgttgatcctcg-3' (SEQ ID NO:13) |
| *streppr10* | 5'-tttggtctcaacactgacttccggcaccaccgagg-3' (SEQ ID NO:14) |
| *streppr11:* | 5'-tttggtctcatctaagtgtggacttccaggcgttggc-3' (SEQ ID NO:15) |
| *streppr12:* | 5'-tttgaagactatagaggacacgacaccttcaccaag-3' (SEQ ID NO:16) |
| *streptag5:* | 5'-ctagcttggagtcatccacagttcgagaaataa-3' (SEQ ID NO:17) |
| *streptag6* | 5'-aagcttatttctcgaactgtggatgactccaag-3' (SEQ ID NO:18) |

## Claims

1. A process of purifying a protein of interest that is an immunoglobulin, comprising the following steps:
(a) providing an affinity matrix of recombinant viral particles or recombinant virus-like particles comprising a fusion protein comprising the following fusion protein domains:
(i) a plant viral coat protein,
(ii) a recombinant protein comprising at least 50 amino acid residues, said recombinant protein being protein A or a domain thereof, or a variant of protein A capable of binding immunoglobulins via the Fc region of the immunoglobulins, or
protein G or a variant of protein G capable of binding immunoglobulins via the Fc region of the immunoglobulins, and
(iii) at least one peptide linker linking said plant viral coat protein and said recombinant protein and comprising at least 10 amino acid residues,
(b) contacting said affinity matrix with a liquid composition containing said protein of interest under conditions allowing binding of said protein of interest to said recombinant protein of said affinity matrix, whereby said affinity matrix is insoluble under said conditions,
(c) removing components of said liquid composition that have not bound to said recombinant protein from the mixture of step (b) under conditions preserving binding of said protein of interest to said recombinant protein of said affinity matrix, and
(d) separating said protein of interest from said affinity matrix.

2. The process according to claim 1, wherein said recombinant protein is protein A or a domain thereof, or a variant of protein A capable of binding immunoglobulins via the Fc region of the immunoglobulins.

3. The process according to any one of claims 1 or 2, said affinity matrix containing at most 20 mol-% of free viral coat protein relative to the sum of free viral coat protein and said fusion protein present in said affinity matrix.

4. Recombinant viral particles or recombinant virus-like particles comprising a plurality of fusion protein molecules, said fusion protein comprising the following fusion protein domains:
(i) a plant viral coat protein,
(ii) a recombinant protein comprising at least 50 amino acid residues,
said recombinant protein being protein A or a domain thereof, or a variant of protein A capable of binding immunoglobulins via the Fc region of the immunoglobulins, or
protein G or a variant of protein G capable of binding immunoglobulins via the Fc region of the immunoglobulins,
and
(iii) a peptide linker linking said plant viral coat protein and said recombinant protein and comprising at least 10 amino acid residues.

5. Recombinant viral particles or recombinant virus-like particles comprising fusion protein molecules, said fusion protein comprising the following fusion protein segments:
(i) a plant viral coat protein,
(ii) a recombinant protein comprising at least 50 amino acid residues,
said recombinant protein being protein A or a domain thereof, or a variant of protein A capable of binding immunoglobulins via the Fc region of the immunoglobulins, or
protein G or a variant of protein G capable of binding immunoglobulins via the Fc region of the immunoglobulins,
and
(iii) a peptide linker linking said plant viral coat protein and said recombinant protein, said peptide linker comprising at least 10 amino acid residues,
wherein said viral particle comprises at most 20 mol-% of free viral coat protein.

6. The viral particles or virus-like particles according to any one of claims 4 or 5, wherein said viral particles are rod-shaped.

7. The viral particles or virus-like particles according to any of claims 4 or 5, wherein said viral coat protein is the coat protein of turnip vein clearing virus or a protein having an identity of at least 60 % to the coat protein of turnip vein clearing virus.

8. The viral particles or virus-like particles according to any one of claims 4 to 6, wherein said plant viral coat protein is tobacco mosaic virus coat protein or a protein having an identity of at least 80 % to the coat protein of tobacco mosaic virus.

9. The viral particles or virus-like particles according to any of claims 4 to 8, wherein said recombinant protein is connected via said peptide linker to the N-terminal end of said plant viral coat protein.

10. The viral particles or virus-like particles according to any of claims 4 to 8, wherein said recombinant protein is connected via said peptide linker to the C-terminal end of said plant viral coat protein.

11. The viral particles or virus-like particles according to any of claims 4 to 10, wherein said peptide linker has no defined secondary structure or forms a helix.

12. The viral particles or virus-like particles according to any of claims 4 to 11, wherein said viral particles display two or more different recombinant proteins on their surface.

13. The viral particles or virus-like particles of claim 4, wherein viral particles comprise at most 20 mol-%, preferably at most 10 mol-%, of free viral coat protein.

14. The viral particles or virus-like particles of claim 5, wherein viral particles comprise at most 10 mol-% of free viral coat protein.

15. The viral particles or virus-like particles according to claim 4 or 5, wherein said plant viral coat protein is a plant viral coat protein of turnip vein clearing virus or a protein having an identity of at least 40 % to the coat protein of turnip vein clearing virus.

16. A process of producing recombinant viral particles or recombinant virus-like particles as defined in any of claims 4 to 15, comprising expressing said fusion protein in a plant, In plant tissue, or in plant cells, said fusion protein comprising a peptide linker of at least 10 amino acid residues linking said plant viral coat protein and said recombinant protein.

17. The process according to claim 16, comprising introducing a plant viral vector encoding said fusion protein into a plant cell by Agrobacterium-mediated delivery, followed by isolating said viral particles from said plant cell.

18. The process according to claim 16 or 17, further comprising rendering said viral particles infection-deficient using chemical inactivation.

19. A process of producing recombinant viral particles or recombinant virus-like particles, comprising assembling recombinant viral particles in a mixture comprising fusion protein as defined in any of claims 4 to 15 and a second protein being or comprising a coat protein under conditions allowing assembly of viral particles comprising said fusion protein and said second protein.

20. Use of a fusion protein comprising the following fusion protein segments: a plant viral coat protein, a linker peptide comprising at least 10 amino acid residues, and a recombinant protein comprising at least 50 amino acid residues,
said recombinant protein being protein A or a domain thereof, or a variant of protein A capable of binding immunoglobulins via the Fc region of the immunoglobulins, or
protein G or a variant of protein G capable of binding immunoglobulins via the Fc region of the immunoglobulins,
for purifying an immunoglobulin.

21. A fusion protein comprising the following fusion protein segments:
(i) a plant viral coat protein,
(ii) protein A or protein G, or variants of protein A or protein G capable of binding to the Fc region of immunoglobulins, and
(iii) a linker peptide comprising at least 10 amino acid residues linking the segments of item (i) and item (ii),
said linker being a flexible linker including one or more proline residues, or having a large proportion of glycine residues and being of sequence (GGGGS)ₙ, n being from 2 to 5;
or said linker is a helix-forming peptide linker of sequence (EAAAK)ₙ, n being from 2 to 5.

22. A polynucleotide encoding the fusion protein defined in any of claims 4 to 15, or 21.

23. A plant, plant tissue or plant cells comprising the polynucleotide of claim 22.

24. Kit-of-parts comprising a plant, plant tissue or plant cells, and a polynucleotide as defined in claim 22.

25. The process according to any one of claims 1 to 3, wherein said fusion protein is as defined in any one of claims 3 to 15.

26. Use of
the fusion protein as defined in any of claims 1 to 15 or 21; or
the recombinant viral particles or virus-like particles as defined in any of claims 4 to 15
for purifying a protein of interest.

## Patentansprüche

1. Verfahren zur Reinigung eines gewünschten Proteins, das ein Immunoglobulin ist, umfassend die folgenden Schritte:
(a) Bereitstellen einer Affinitätsmatrix aus rekombinanten viralen Partikeln oder rekombinanten virusähnlichen Partikeln umfassend ein Fusionsprotein umfassend die folgenden Fusionsproteindomänen:
(i) ein pflanzenvirales Hüllprotein,
(ii) ein rekombinantes Protein umfassend mindestens 50 Aminosäurereste, wobei das rekombinante Protein Protein A oder eine Domäne desselben ist oder eine Variante von Protein A, die an Immunoglobuline über die Fc-Region der Immunoglobuline binden kann, oder
Protein G oder eine Variante von Protein G, die an Immunoglobuline über die Fc-Region der Immunoglobuline binden kann, und
(iii) mindestens einen Peptidlinker, der das pflanzenvirale Hüllprotein und das rekombinante Protein verbindet und mindestens 10 Aminosäurereste umfasst,
(b) Kontaktieren der Affinitätsmatrix mit einer flüssigen Zusammensetzung enthaltend das gewünschte Protein unter Bedingungen, die ein Binden des gewünschten Proteins an das rekombinante Protein der Affinitätsmatrix erlauben, wobei die Affiinitätsmatrix unter diesen Bedingungen unlöslich ist,
(c) Entfernen von Komponenten aus der flüssigen Zusammensetzung, die nicht an das rekombinante Protein gebunden haben, aus der Mischung von Schritt (b) unter Bedingungen, bei denen die Bindung des gewünschten Proteins an das rekombinante Protein der Affinitätsmatrix erhalten bleibt, und
(d) Abtrennen des gewünschten Proteins von der Affinitätsmatrix.

2. Das Verfahren gemäß Anspruch 1, wobei das rekombinante Protein Protein A ist oder eine Domäne davon, oder eine Variante von Protein A, die an Immunoglobuline über deren Fc-Region binden kann.

3. Das Verfahren gemäß einem der Ansprüche 1 oder 2, wobei die Affinitätsmatrix höchstens 20 mol-% an freiem viralen Hüllprotein bezogen auf die Summe aus freiem viralen Hüllprotein und dem Fusionsprotein in der Affinitätsmatrix enthält.

4. Rekombinante virale Partikel oder rekombinante virusähnliche Partikel umfassend eine Vielzahl an Fusionsproteinmolekülen, wobei das Fusionsprotein die folgenden Fusionsproteindomänen enthält:
(i) ein pflanzenvirales Hüllprotein,
(ii) ein rekombinantes Protein enthaltend mindestens 50 Aminosäurereste, wobei das rekombinante Protein Protein A oder eine Domäne davon ist, oder eine Variante von Protein A, die an Immunoglobuline über die Fc-Region der Immunoglobuline binden kann, oder
Protein G oder eine Variante von Protein G, die an Immunoglobuline über die Fc-Region der Immunoglobuline binden kann,
und
(iii) einen Peptidlinker, der das pflanzenvirale Hüllprotein und das rekombinante Protein verbindet und mindestens 10 Aminosäurereste umfasst.

5. Rekombinante virale Partikel oder rekombinante virusähnliche Partikel, umfassend Fusionsprotein-Moleküle, wobei das Fusionsprotein die folgenden Fusionsproteinsegmente aufweist:
(i) ein pflanzenvirales Hüllprotein,
(ii) ein rekombinantes Protein enthaltend mindestens 50 Aminosäurereste, wobei das rekombinante Protein Protein A oder eine Domäne davon ist, oder eine Variante von Protein A, die an Immunoglobuline über die Fc-Region der Immunoglobuline binden kann, oder
Protein G oder eine Variante von Protein G, die an Immunoglobuline über die Fc-Region der Immunoglobuline binden kann,
und
(iii) einen Peptidlinker, der das pflanzenvirale Hüllprotein und das rekombinante Protein verbindet und mindestens 10 Aminosäurereste umfasst,
wobei das virale Partikel höchstens 20 mol-% an freiem viralen Hüllprotein enthält.

6. Die viralen Partikel oder virusähnlichen Partikel gemäß einem der Ansprüche 4 oder 5, worin die viralen Partikel stäbchenförmig sind.

7. Die viralen Partikel oder virusähnlichen Partikel gemäß einem der Ansprüche 4 oder 5, worin das virale Hüllprotein das Hüllprotein von turnip vein clearing virus oder ein Protein mit einer Identität von mindestens 60% zum Hüllprotein des turnip vein clearing virus ist.

8. Die viralen Partikel oder virusähnlichen Partikel gemäß einem der Ansprüche 4 bis 6, worin das pflanzenvirale Hüllprotein das tobacco mosaic virus Hüllprotein ist oder ein Protein mit einer Identität von mindestens 80% zum Hüllprotein des tobacco mosaic virus.

9. Die viralen Partikel oder virusähnlichen Partikel gemäß einem der Ansprüche 4 bis 8, worin das rekombinante Protein über den Peptidlinker an das N-terminale Ende des pflanzenviralen Hüllproteins gebunden ist.

10. Die viralen Partikel oder virusähnlichen Partikel gemäß einem der Ansprüche 4 bis 8, worin das rekombinante Protein über den Peptidlinker an das C-terminale Ende des pflanzenviralen Hüllproteins gebunden ist.

11. Die viralen Partikel oder virusähnlichen Partikel gemäß einem der Ansprüche 4 bis 10, worin der Peptidlinker keine definierte Sekundärstruktur aufweist oder eine Helix bildet.

12. Die viralen Partikel oder virusähnlichen Partikel gemäß einem der Ansprüche 4 bis 11, worin die viralen Partikel zwei oder mehr verschiedene rekombinante Proteine auf ihrer Oberfläche präsentieren.

13. Die viralen Partikel oder virusähnlichen Partikel gemäß Anspruch 4, worin die viralen Partikel höchstens 20 mol-%, vorzugsweise höchstens 10 mol-% an freiem viralen Hüllprotein enthalten.

14. Die viralen Partikel oder virusähnlichen Partikel gemäß Anspruch 5, worin die viralen Partikel höchstens 10 mol-% an freiem viralen Hüllprotein enthalten.

15. Die viralen Partikel oder virusähnlichen Partikel gemäß Anspruch 4 oder 5, worin das pflanzenvirale Hüllprotein ein pflanzenvirales Hüllprotein von turnip vein clearing virus ist oder ein Protein mit einer Identität von mindestens 40% zum Hüllprotein des turnip vein clearing virus.

16. Verfahren zur Herstellung von rekombinanten viralen Partikeln oder rekombinanten virusähnlichen Partikeln wie in einem der Ansprüche 4 bis 15 definiert, umfassend Exprimieren des Fusionsproteins in einer Pflanze, in Pflanzengewebe oder in Pflanzenzellen, wobei das Fusionsprotein einen Peptidlinker aus mindestens 10 Aminosäureresten enthält, der das pflanzenvirale Protein und das rekombinante Protein miteinander verbindet.

17. Das Verfahren gemäß Anspruch 16, umfassend Agrobakterium-vermitteltes Einführen eines pflanzenviralen Vektors, der für das Fusionsprotein kodiert, in eine Pflanzenzelle, gefolgt von Isolieren der viralen Partikel aus der Pflanzenzelle.

18. Das Verfahren gemäß Anspruch 16 oder 17, wobei die viralen Partikel infektionsunfähig gemacht werden mittels chemischer Inaktivierung.

19. Verfahren zur Herstellung von rekombinanten viralen Partikeln oder rekombinanten virusähnlichen Partikeln, umfassend ein Aufbauen von rekombinanten viralen Partikeln in einer Mischung enthaltend das Fusionsprotein wie in einem der Ansprüche 4 bis 15 definiert und ein zweites Protein, das ein Hüllprotein ist oder umfasst, unter Bedingungen, die den Aufbau von viralen Partikeln umfassend das Fusionsprotein und das zweite Protein erlauben.

20. Verwendung eines Fusionsproteins umfassend die folgenden Fusionsproteinsegmente: ein pflanzenvirales Hüllprotein, ein Linkerpeptid umfassend mindestens 10 Aminosäurereste, und ein rekombinantes Protein umfassend mindestens 50 Aminosäurereste,
wobei das rekombinante Protein Protein A oder eine Domäne davon ist oder eine Variante von Protein A, die an Immunoglobuline über die Fc-Region der Immunoglobuline binden kann, oder
Protein G oder eine Variante von Protein G, die an Immunoglobuline über die Fc-Region der Immunoglobuline binden kann,
zur Reinigung eines Immunoglobulins.

21. Fusionsprotein umfassend die folgenden Fusionsproteinsegmente:
(i) ein pflanzenvirales Hüllprotein,
(ii) Protein A oder Protein G, oder Varianten von Protein A oder Protein G, die an die Fc-Region von Immunoglobulinen binden können, und
(iii) ein Linkerpeptid umfassend mindestens 10 Aminosäurereste, das die Segmente von Punkt (i) und Punkt (ii) miteinander verbindet,
wobei der Linker ein flexibler Linker ist, der ein oder mehrere Prolinreste aufweist, oder der einen großen Anteil an Glycinresten aufweist und die Sequenz (GGGGS)ₙ hat, wobei n 2 bis 5 ist;
oder der Linker ein Helix-bildender Peptidlinker der Sequenz (EAAAK)ₙ ist, wobei n 2 bis 5 ist.

22. Ein Polynukleotid, das für das Fusionsprotein wie in einem der Ansprüche 4 bis 15 oder 21 definiert kodiert.

23. Pflanze, Pflanzengewebe oder Pflanzenzellen umfassend das Polynukleotid von Anspruch 22.

24. Kit umfassend eine Pflanze, Pflanzengewebe oder Pflanzenzellen und ein Polynukleotid wie in Anspruch 22 definiert.

25. Das Verfahren gemäß einem der Ansprüche 1 bis 3, wobei das Fusionsprotein wie in einem der Ansprüche 3 bis 15 definiert ist.

26. Verwendung
des Fusionsproteins wie in einem der Ansprüche 1 bis 15 oder 21 definiert; oder
der rekombinanten viralen Partikel oder virusähnlichen Partikel wie in einem der Ansprüche 4 bis 15 definiert
zur Reinigung eines gewünschten Proteins.

## Revendications

1. Procédé pour purifier une protéine présentant un intérêt qui est une immunoglobuline, comprenant les étapes suivantes consistant à :
(a) se procurer une matrice d'affinité de particules virales recombinées ou de particules analogues à un virus recombinées comprenant une protéine de fusion comprenant les domaines de protéine de fusion suivantes :
(i) une protéine d'enveloppe de virus de plante ;
(ii) une protéine recombinée comprenant au moins 50 résidus d'acides aminés, ladite protéine recombinée étant la protéine A ou un domaine de celle-ci, ou un variant de protéine A capable de se lier à des immunoglobulines via la région Fc des immunoglobulines, ou
la protéine G ou un variant de protéine G capable de se lier à des immunoglobulines via la région Fc des immunoglobulines, et
(iii) au moins un lieur peptidique liant ladite protéine d'enveloppe de virus de plante et ladite protéine recombinée et comprenant au moins 10 résidus d'acides aminés,
(b) mettre en contact ladite matrice d'affinité avec une composition liquide contenant ladite protéine présentant un intérêt dans des conditions permettant la liaison de ladite protéine présentant un intérêt à ladite protéine recombinée de ladite matrice d'affinité, ladite matrice d'affinité étant ainsi insoluble dans lesdites conditions,
(c) éliminer les composants dans ladite composition liquide qui ne se sont pas liés à ladite protéine recombinée dans le mélange de l'étape (b) dans des conditions préservant la liaison de ladite protéine présentant un intérêt à ladite protéine recombinée de ladite matrice d'affinité, et
(d) séparer ladite protéine présentant un intérêt d'avec ladite matrice d'affinité.

2. Procédé selon la revendication 1, dans lequel ladite protéine recombinée est la protéine A ou un domaine de celle-ci, ou un variant de protéine A capable de se lier à des immunoglobulines via la région Fc des immunoglobulines.

3. Procédé selon l'une quelconque des revendications 1 et 2, dans lequel ladite matrice d'affinité contient au plus 20 % en moles de protéine d'enveloppe virale libre par rapport à la somme de la protéine d'enveloppe virale libre et de ladite protéine de fusion présente dans ladite matrice d'affinité.

4. Particules virales recombinées ou particules analogues à un virus recombinées comprenant une pluralité de molécules de protéine de fusion, ladite protéine de fusion comprenant les domaines de protéine de fusion suivantes :
(i) une protéine d'enveloppe de virus de plante ;
(ii) une protéine recombinée comprenant au moins 50 résidus d'acides aminés, ladite protéine recombinée étant la protéine A ou un domaine de celle-ci, ou un variant de protéine A capable de se lier à des immunoglobulines via la région Fc des immunoglobulines, ou
la protéine G ou un variant de protéine G capable de se lier à des immunoglobulines via la région Fc des immunoglobulines, et
(iii) un lieur peptidique liant ladite protéine d'enveloppe de virus de plante et ladite protéine recombinée et comprenant au moins 10 résidus d'acides aminés.

5. Particules virales recombinées ou particules analogues à un virus recombinées comprenant des molécules de protéine de fusion, ladite protéine de fusion comprenant les segments de protéine de fusion suivants :
(i) une protéine d'enveloppe de virus de plante ;
(ii) une protéine recombinée comprenant au moins 50 résidus d'acides aminés, ladite protéine recombinée étant la protéine A ou un domaine de celle-ci, ou un variant de protéine A capable de se lier à des immunoglobulines via la région Fc des immunoglobulines, ou
la protéine G ou un variant de protéine G capable de se lier à des immunoglobulines via la région Fc des immunoglobulines, et
(iii) un lieur peptidique liant ladite protéine d'enveloppe de virus de plante et ladite protéine recombinée, ledit lieur peptidique comprenant au moins 10 résidus d'acides aminés,
où ladite particule virale comprend au plus 20 % en moles de protéine d'enveloppe virale libre.

6. Particules virales ou particules analogues à un virus selon l'une quelconque des revendications 4 et 5, lesquelles particules virales sont en forme de bâtonnet.

7. Particules virales ou particules analogues à un virus selon l'une quelconque des revendications 4 et 5, dans lesquelles ladite protéine d'enveloppe virale est la protéine d'enveloppe du virus de la décoloration des nervures du navet ou une protéine présentant une identité d'au moins 60 % avec la protéine d'enveloppe du virus de la décoloration des nervures du navet.

8. Particules virales ou particules analogues à un virus selon l'une quelconque des revendications 4 à 6, dans lesquelles ladite protéine d'enveloppe de virus de plante est la protéine d'enveloppe du virus de la mosaïque du tabac ou une protéine présentant une identité d'au moins 80 % avec la protéine d'enveloppe du virus de la mosaïque du tabac.

9. Particules virales ou particules analogues à un virus selon l'une quelconque des revendications 4 à 8, dans lesquelles ladite protéine recombinée est connectée via ledit lieur peptidique à l'extrémité N-terminale de ladite protéine d'enveloppe de virus de plante.

10. Particules virales ou particules analogues à un virus selon l'une quelconque des revendications 4 à 8, dans lesquelles ladite protéine recombinée est connectée via ledit lieur peptidique à l'extrémité C-terminale de ladite protéine d'enveloppe de virus de plante.

11. Particules virales ou particules analogues à un virus selon l'une quelconque des revendications 4 à 10, dans lesquelles ledit lieur peptidique ne présente pas de structure secondaire définie ou forme une hélice.

12. Particules virales ou particules analogues à un virus selon l'une quelconque des revendications 4 à 11, lesquelles particules virales présentent deux ou plus de deux protéines recombinées différentes sur leur surface.

13. Particules virales ou particules analogues à un virus selon la revendication 4, lesquelles particules virales comprennent au plus 20 % en moles et de préférence au plus 10 % en moles de protéine d'enveloppe virale libre.

14. Particules virales ou particules analogues à un virus selon la revendication 5, lesquelles particules virales comprennent au plus 10 % en moles de protéine d'enveloppe virale libre.

15. Particules virales ou particules analogues à un virus selon la revendication 4 ou 5, dans lesquelles ladite protéine d'enveloppe de virus de plante est une protéine d'enveloppe de virus de plante du virus de la décoloration des nervures du navet ou une protéine présentant une identité d'au moins 40 % avec la protéine d'enveloppe du virus de la décoloration des nervures du navet.

16. Procédé pour produire des particules virales recombinées ou des particules analogues à un virus recombinées telles que définies dans l'une quelconque des revendications 4 à 15, comprenant l'expression de ladite protéine de fusion dans une plante, dans un tissu de plante, ou dans des cellules de plante, ladite protéine de fusion comprenant un lieur peptidique d'au moins 10 résidus d'acides aminés liant ladite protéine d'enveloppe de virus de plante et ladite protéine recombinée.

17. Procédé selon la revendication 16, comprenant l'introduction d'un vecteur de type virus de plante codant ladite protéine de fusion dans une cellule de plante par délivrance à médiation par Agrobacterium, suivie de l'isolation desdites particules virales à partir de ladite cellule de plante.

18. Procédé selon la revendication 16 ou 17, comprenant en outre le fait de rendre lesdites particules virales inefficaces pour une infection par utilisation d'une inactivation chimique.

19. Procédé pour produire des particules virales recombinées ou des particules analogues à un virus recombinées, comprenant l'assemblage de particules virales recombinées dans un mélange comprenant une protéine de fusion telle que définie dans l'une quelconque des revendications 4 à 15 et une deuxième protéine qui est ou qui comprend une protéine d'enveloppe, dans des conditions permettant l'assemblage de particules virales comprenant ladite protéine de fusion et ladite deuxième protéine.

20. Utilisation d'une protéine de fusion comprenant les segments de protéine de fusion suivants : une protéine d'enveloppe de virus de plante, un peptide lieur comprenant au moins 10 résidus d'acides aminés, et une protéine recombinée comprenant au moins 50 résidus d'acides aminés,
ladite protéine recombinée étant la protéine A ou un domaine de celle-ci, ou un variant de protéine A capable de se lier à des immunoglobulines via la région Fc des immunoglobulines, ou
la protéine G ou un variant de protéine G capable de se lier à des immunoglobulines via la région Fc des immunoglobulines,
pour la purification d'une immunoglobuline.

21. Protéine de fusion comprenant les segments de protéine de fusion suivantes :
(i) une protéine d'enveloppe de virus de plante,
(ii) la protéine A ou la protéine G, ou des variants de protéine A ou de protéine G capables de se lier à la région Fc d'immunoglobulines, et
(iii) un peptide lieur comprenant au moins 10 résidus d'acides aminés liant les segments du paragraphe (i) et du paragraphe (ii),
ledit lieur étant un lieur flexible contenant un ou plusieurs résidus de proline, ou présentant une forte proportion de résidus de glycine et comportant la séquence (GGGGS)ₙ, où n vaut de 2 à 5 ;
ou ledit lieur étant un lieur peptidique formant une hélice ayant la séquence (EAAAK)ₙ, où n vaut de 2 à 5.

22. Polynucléotide codant la protéine de fusion définie dans l'une quelconque des revendications 4 à 15 et 21.

23. Plante, tissu de plante ou cellules de plante comprenant le polynucléotide de la revendication 22.

24. Kit de pièces comprenant une plante, un tissu de plante ou des cellules de plante, et un polynucléotide tel que défini dans la revendication 22.

25. Procédé selon l'une quelconque des revendications 1 à 3, dans lequel ladite protéine de fusion est telle que définie dans l'une quelconque des revendications 3 à 15.

26. Utilisation de
la protéine de fusion telle que définie dans l'une quelconque des revendications 1 à 15 et 21 ; ou
les particules virales recombinées ou les particules analogues à un virus telles que définies dans l'une quelconque des revendications 4 à 15
pour la purification d'une protéine présentant un intérêt.
